# EUROPEAN PATENT SPECIFICATION

(11) **EP 3 310 168 B1**
(45) Date of publication and mention of the grant of the patent: **21.08.2024**
(21) Application number: 16812548.2
(22) Date of filing: 17.06.2016
(51) Int. Cl.: A61K 31/4406, A61K 31/522, A61K 31/4155, A61K 45/06, A61P 37/02, A61P 29/00, A61P 35/00, A61P 31/22

(54) **COMBINATIONS OF A NUCLEOSIDE ANALOG AND AN INDUCING AGENT FOR USE IN TREATING HERPESVIRUS-INDUCED CONDITIONS**
KOMBINATIONEN AUS EINEM NUKLEOSID-ANALOGON UND EINEM INDUZIERENDEN MITTEL ZUR VERWENDUNG BEI DER BEHANDLUNG VON HERPESVIRUS-INDUZIERTEN ZUSTÄNDEN
COMBINAISONS D'UN ANALOGUE DE NUCLEOSIDE ET D'UN AGENT INDUCTEUR POUR LE TRAITEMENT DES CONDITIONS INDUITES PAR LE VIRUS HERPÉTIQUE

(30) Priority: 19.06.2015 US 201562182071 P
(43) Date of publication of application: 25.04.2018
(73) Proprietor: Trustees of Boston University, Boston, MA 02215 (US)
(72) Inventor: FALLER, Douglas V., Weston, Massachusetts 02493 (US)
(74) Representative: HGF
(86) International application number: PCT/US2016/038148
(87) International publication number: WO 2016/205695

(56) References cited:
- CN-A- 104 083 763
- CN-A- 104 083 763
- US-A1- 2013 331 313
- US-A1- 2013 331 313
- US-A1- 2014 045 774
- US-A1- 2014 045 774
- US-A1- 2014 170 221
- US-A1- 2014 170 221
- US-A1- 2014 341 989
- SAJAL K GHOSH ET AL: "Histone deacetylase inhibitors are potent inducers of gene expression in latent EBV and sensitize lymphoma cells to nucleoside antiviral agents", BLOOD, 7 December 2011 (2011-12-07), pages 1008 - 1017, XP055548340, Retrieved from the Internet <URL:http://www.bloodjournal.org/content/bloodjournal/119/4/1008.full.pdf> [retrieved on 20190128], DOI: 10.1182/blood-2011-06-
- GHOSH ET AL: "Short, discontinuous exposure to butyrate effectively sensitizes latently EBV-infected lymphoma cells to nucleoside analogue antiviral agents", BLOOD CELLS, MOLECULES & DISEASES, LAJOLLA, US, vol. 38, no. 1, 23 December 2006 (2006-12-23), pages 57 - 65, XP005733333, ISSN: 1079-9796, DOI: 10.1016/J.BCMD.2006.10.008

## Description

The present invention relates to an inducing agent in combination with an antiviral agent, for use in a method for treating or preventing a cancer, autoimmune, or inflammatory condition caused by herpesvirus infection in a subject having a latent herpesvirus infection, as defined in the claims.

### BACKGROUND OF THE INVENTION

Herpesvirus can induce autoimmune or inflammatory conditions through a number of potential mechanisms, e.g.; 1) activating B cells to produce auto-antibodies, 2) activating T cells that attack host tissue, 3) molecular mimicry in which herpesvirus antigens cross react with host antigens, such that an autoimmune condition results when T cells or antibodies that are reactive with these antigens cross react with host antigens causing damage to host tissues, 4) herpesvirus infected cells produce cytokines which turn on other elements of the immune system and increase inflammation which can also exacerbate autoimmune conditions. Autoimmune or inflammatory conditions with evidence of a herpesvirus relationship include multiple sclerosis, systemic lupus erythematosus, rheumatoid arthritis and Sjögren's syndrome, hepatitis, colitis, retinitis, pneumonitis, esophagitis, transverse myelitis, encephalitis or polyradiculopathy. Cytomegalovirus and herpes simplex virus have also been associated with coronary artery disease.

A number of cancers and neoplasias have been associated with various herpesviruses including but not limited to mucoepidermoid carcinoma, lymphomas, Burkitt's lymphoma, Hodgkin's lymphoma, nasopharyngeal carcinoma and lymphomatoid granulomatosis, and cervical cancer.

US 2013331313 discloses methods and compositions for treating viral or virally-induced conditions.

Sajal K Ghosh *et al.* discloses that histone deacetylase inhibitors are potent inducers of gene expression in latent EBV and sensitize lymphoma cells to nucleoside antiviral agents (Blood, 2012 Jan 26;119(4):1008-17).

US 2014045774 discloses methods for treating viral disorders.

### SUMMARY OF THE INVENTION

The references to methods of treatment in this description are to be interpreted as references to the compounds, pharmaceutical compositions and medicaments of the present invention for use in a method for treatment of the human (or animal) body by therapy (or for diagnosis).

The invention is defined by the claims. Further aspects and preferred embodiments are defined in the dependent claims. Any aspects, embodiments and examples of the present disclosure which do not fall under the scope of the appended claims do not form part of the invention and are merely provided for illustrative purposes.

The invention provides an inducing agent in combination with an antiviral agent, for use in a method for treating or preventing a cancer, autoimmune, or inflammatory condition caused by herpesvirus infection in a subject, wherein said antiviral agent is a nucleoside analog and said inducing agent is chidamide in accordance with the claims.

In one embodiment, the invention provides a method for treating or preventing a condition caused by herpesvirus infection in a subject, comprising administering to the subject an inducing agent to induce expression of a viral gene product in a virus-infected cell of the subject, wherein the antiviral agent is a nucleoside analog, and an anti-viral agent whose anti-viral activity is directed to the viral gene product expressed, wherein said inducing agent is an HDAC inhibitor, wherein the HDAC inhibitor is Chidamide.

In certain embodiments, the condition is a cancer ·caused by Epstein-Barr virus infection. In certain further embodiments, the cancer is lymphoma, Burkitt's Lymphoma, Hodgkin's disease, nasopharyngeal carcinoma, hairy leukoplakia, NK/T cell lymphoma or a lymphoma of the central nervous system. In certain other embodiments, the condition is an autoimmune or inflammatory condition caused by Epstein-Barr Virus infection: dermatomyositis,
Sjögren's syndrome, or multiple sclerosis.

In certain embodiments, the condition is a cancer caused by herpes simplex virus infection. In certain embodiments, the condition is a cancer caused by -human herpes virus 8 infection.

In certain embodiments, the condition is a cancer caused by cytomegalovirus infection. In certain further embodiments, the cancer is mucoepidermoid carcinoma. In certain other embodiments, the condition is an inflammatory disease caused by cytomegalovirus infection; hepatitis, colitis, retinitis, pneumonitis, esophagitis, transverse myelitis, encephalitis or polyradiculopathy.

In certain embodiments, the condition is a lymphoma.

In certain embodiments, the inducing agent induces expression of a viral gene product in a virus-infected cell of the subject, wherein the viral gene product is a viral enzyme, an oncogene or proto-oncogene, a transcription factor, a protease, a polymerase, a reverse transcriptase, a cell surface receptor, a structural protein, a major histocompatibility antigen, a growth factor, or a combination thereof. In certain further embodiments, the gene product is thymidine kinase or protein kinase. In certain other embodiments, the inducing agent induces viral TK expression.

In certain embodiments, the inducing agent is administered at a dose of about 1.0 to about 1000 mg/day. In certain embodiments, the inducing agent is administered twice daily (bid).

In certain embodiments, the anti-viral agent is a nucleoside analog. In certain further embodiments, the nucleoside analog is selected from the group consisting of acyclovir (ACV), ganciclovir (GCV), valganciclovir, famcyclovir, penciclovir (PCV), ribavirin, zalcitabine (ddC), zidovudine (AZT), stavudine (D4T), lamivudine (3TC), didanosine (ddl), cytarabine, dideoxyadenosine, edoxudine, floxuridine, idoxuridine, inosine pranobex, 2'-deoxy-5-(methylamino)uridine, trifluridine or vidarabine. In certain embodiments, the antiviral is ganciclovir.

In certain non-claimed aspects of the disclosure, the inducing agent is an HDAC inhibitor having inhibitory activity at a concentration less than or equal to 500 nanomolar. In certain non-claimed aspects, the inducing agent is an HDAC inhibitor capable of inducing thymidine kinase expression at a concentration less than or equal to 500 nanomolar. In certain other embodiments, the plasma level of the inducing agent in said subject is less than 5 µM. In certain embodiments, the inducing agent is administered orally. According to the invention, the inducing agent is chidamide.

In certain embodiments, the inducing agent and anti-viral agent are administered for at least one cycle of therapy, said cycle comprising: (i) administering the inducing agent and the anti-viral agent to the subject over a first period of time; and (ii) continuing the administration of the anti-viral agent without administering the inducing agent to the subject for a second period; wherein said second period represents the remainder of the cycle.

. In certain further embodiments, the first period anti-viral agent and inducing agent are administered in the same composition. In certain embodiments, the first period of time is less than or equal to one-half of the length of the cycle. In certain embodiments, the first period of time is less than or equal to about 5 days, and wherein said cycle is less than or equal to about 21 days. In certain embodiments, the inducing agent is administered for a period of 14 days, followed by a period of 7 days wherein the agent is not administered.

In certain embodiments, the virally associated condition is not sepsis or viremia.

### BRIEF DESCRIPTION OF THE DRAWINGS

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

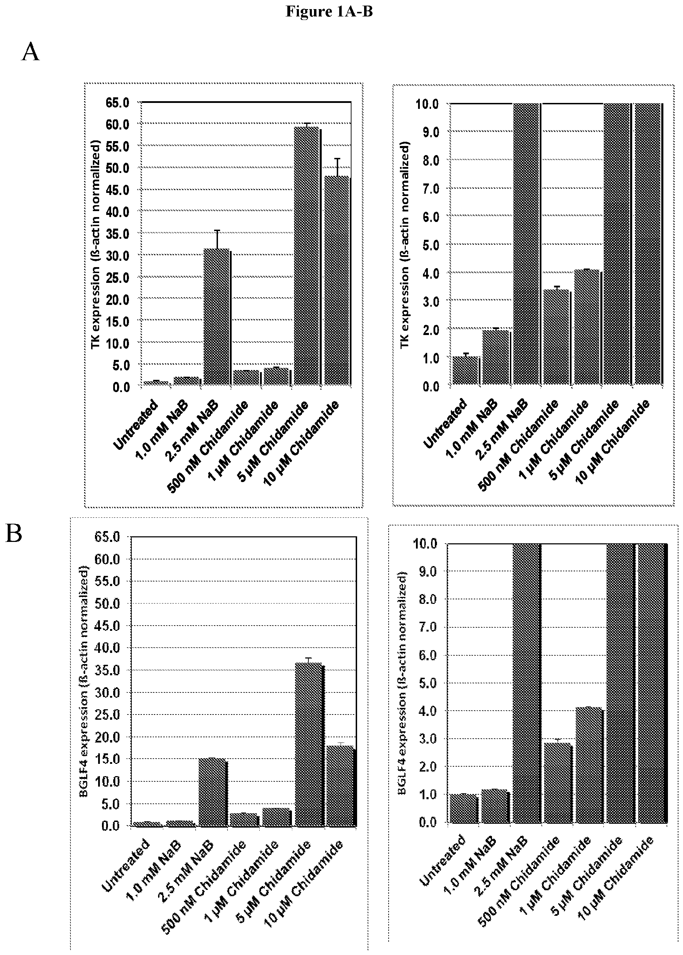

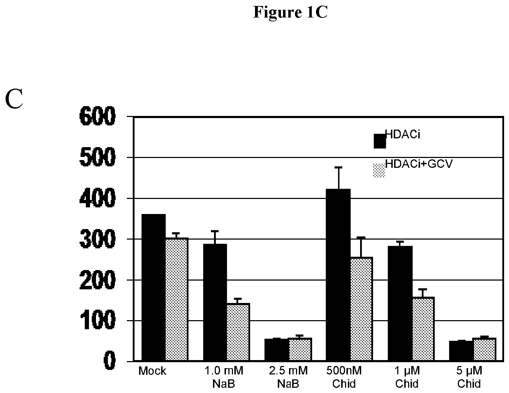

**Figure** 1 illustrates results from tests for the efficacy of chidamide in inducing expression of viral kinase transcripts in the EBV+ cell line P3HR1 for TK (Figure 1A) and BGLF4 **(****Figure 1B****).** **Figure 1C** illustrates the synergistic effect that chidamide has on killing of the EBV+ cell line P3HR1 when combined with ganciclovir.

### DETAILED DESCRIPTION OF THE INVENTION

There is a need for methods of treating and/or preventing viral conditions, viral-induced conditions, and related cancers and inflammatory conditions. Many patients have latent infections in which a virus is present, but is not expressing viral proteins such as viral thymidine kinase or protein kinase, the target for common anti-viral drugs such as acyclovir, ganciclovir, and valganciclovir. A virus-inducing drug such as a histone deacetylase inhibitor (HDAC inhibitor - HDACi) can be used to re-induce the expression of viral thymidine kinase or protein kinase in virus infected cells in the subject; the subject can then be treated with antiviral drugs to eliminate latent viral infections. As herpesvirus and/or other latent viral infections can be associated with a variety of conditions, including autoimmune and inflammatory conditions, cancers, and allergic conditions, eliminating the latent virus with HDACi therapy is useful in preventing or treating such conditions.

Provided but not claimed herein are methods and compositions for treating and/or preventing viral diseases and conditions, cancers or inflammatory diseases and conditions in a subject. According to the invention, the condition is caused by herpesvirus infection in a subject having latent viral infection in accordance with the claims.

The method can comprise the steps of administering a viral inducing agent and an antiviral agent to the subject. The method can comprise steps of administering a viral inducing agent, an antiviral agent, and one or more additional agents to a subject. The methods may include the co-administration of an oral HDAC inhibitor and an antiviral agent, either in the same or separate formulations.

The methods and compositions provided can be used to treat and/or prevent infection by any of the viruses described herein. The methods and compositions can be used to treat and/or prevent any of the cancers described herein. The methods and compositions can be used to treat and/or prevent any of the inflammatory conditions described herein. Any of the viral inducing agents and/or antiviral agents described herein can be used in the methods and compositions disclosed but not claimed herein. The viral inducing agent can be an HDAC inhibitor. The HDAC inhibited can be any of a class I HDAC, for instance, HDAC1, HDAC2, and HDAC3. The HDAC inhibited can be a class IIb HDAC, for instance, HDAC10. The HDAC inhibitor can be a benzamide. The benzamide can be 4SC-202. According to the invention, the benzamide is chidamide (also known as CS055 or HBI-8000).

One or more additional agents described herein can be administered to a subject. An additional agent can be selected for administration based on the type of condition the subject has or is suspected of having.

Disclosed but not claimed herein are also formulations, routes of administration and effective doses for pharmaceutical compositions comprising an agent or combination of agents, e.g., viral inducing agents, antiviral agents, or one or more additional agents. A viral inducing agent, antiviral agent, or one or more additional agents can be administered to a subject in separate pharmaceutical compositions or can be co-formulated in a single pharmaceutical composition.

Also provided but not claimed are methods relating to dosing schedules for administering a viral inducing agent, antiviral agent, or one or more additional agents. One or more pharmaceutical compositions can be administered to a subject by "pulsed administration" over a period of time.

### DEFINITIONS

The terms "viral," "virus-associated," and "virally-induced" with reference to disorders are used interchangeably throughout the instant specification.

The term "obtaining" as in "obtaining the composition" is intended to include purchasing, synthesizing, or otherwise acquiring the composition (or agent(s) of the composition).

The terms "comprises", "comprising", are intended to have the broad meaning ascribed to them and can mean "includes", "including" and the like.

The term "subject", "patient" or "individual" are used interchangeably herein and refer to mammals and non-mammals, e.g., suffering from a disorder described herein. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. In one embodiment of the methods and compositions provided herein, the mammal is a human.

The terms "treat," "treating" or "treatment," and other grammatical equivalents as used herein, include alleviating, inhibiting or reducing symptoms, reducing or inhibiting severity of, reducing incidence of, prophylactic treatment of, reducing or inhibiting recurrence of, delaying onset of, delaying recurrence of, abating or ameliorating a disease or condition symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. The terms further include achieving a therapeutic benefit. By therapeutic benefit is meant eradication or amelioration of the underlying disorder being treated, and/or the eradication or amelioration of one or more of the physiological symptoms associated with the underlying disorder such that an improvement is observed in the patient.

The terms "prevent," "preventing" or "prevention," and other grammatical equivalents as used herein, include preventing additional symptoms, preventing the underlying metabolic causes of symptoms, inhibiting the disease or condition, e.g., arresting the development of the disease or condition and are intended to include prophylaxis. The terms further include achieving a prophylactic benefit. For prophylactic benefit, the compositions are optionally administered to a patient at risk of developing a particular disease, to a patient reporting one or more of the physiological symptoms of a disease, or to a patient at risk of reoccurrence of the disease.

The terms "effective amount" or "therapeutically effective amount" as used herein, refer to a sufficient amount of at least one agent being administered which achieve a desired result, e.g., to relieve to some extent one or more symptoms of a disease or condition being treated. In certain instances, the result is a reduction and/or alleviation of the signs, symptoms, or causes of a disease, or any other desired alteration of a biological system. In certain instances, an "effective amount" for therapeutic uses is the amount of the composition comprising an agent as set forth herein required to provide a clinically significant decrease in a disease. An appropriate "effective" amount in any individual case is determined using any suitable technique, such as a dose escalation study.

The terms "administer," "administering", "administration," and the like, as used herein, refer to the methods that are used to enable delivery of agents or compositions to the desired site of biological action. These methods include, but are not limited to oral routes, intraduodenal routes, parenteral injection (including intravenous, subcutaneous, intraperitoneal, intramuscular, intravascular or infusion), topical and rectal administration. Administration techniques that in some instances are employed with the agents and methods described herein include, e.g., as discussed in Goodman and Gilman, The Pharmacological Basis of Therapeutics (current edition), Pergamon; and Remington's, Pharmaceutical Sciences (current edition), Mack Publishing Co., Easton, Pa. In certain embodiments, the agents and compositions described herein are administered orally. In some embodiments, the compositions described herein are administered parenterally.

The term "pharmaceutically acceptable" as used herein, refers to a material that does not abrogate the biological activity or properties of the agents described herein, and is relatively nontoxic (i.e., the toxicity of the material significantly outweighs the benefit of the material). In some instances, a pharmaceutically acceptable material is administered to an individual without causing significant undesirable biological effects or significantly interacting in a deleterious manner with any of the components of the composition in which it is contained.

The term "pharmaceutically acceptable excipient," as used herein, refers to carriers and vehicles that are compatible with the active ingredient (for example, a compound disclosed herein) of a pharmaceutical composition disclosed herein (and preferably capable of stabilizing it) and not deleterious to the subject to be treated. For example, solubilizing agents that form specific, more soluble complexes with the compounds of the invention can be utilized as pharmaceutical excipients for delivery of the compounds. Suitable carriers and vehicles are known to those of extraordinary skill in the art. The term "excipient" as used herein will encompass all such carriers, adjuvants, diluents, solvents, or other inactive additives. Suitable pharmaceutically acceptable excipients include, but are not limited to, water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethyl-cellulose, polyvinylpyrrolidone, etc. The pharmaceutical compositions disclosed herein can also be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like, which do not deleteriously react with the active compounds disclosed herein.

The invention can be understood more fully by reference to the following detailed description and illustrative examples, which are intended to exemplify embodiments of the invention.

### HERPESVIRUSES

Herpesviruses are a large family of DNA viruses that include Herpes simplex viruses (HSVs) 1 and 2, Varicella zoster virus, Epstein-Barr virus (EBV), cytomegalovirus (CMV) and human herpesviruses (HHVs) 6A, 6B, 7 and 8, which can cause various diseases in humans. Herpesviruses have two stages of replication, the lytic and the latent. Soon after primary infection, immunological surveillance by the host force herpesviruses to enter the latent state of infection, where only a few selected genes are expressed. Conventional anti-herpesvirus drugs, such as ganciclovir, acyclovir, etc., fail to act on these latently-infected cells because the viral enzyme thymidine kinase (TK) or protein kinase (PK), which is necessary for the conversion of the prodrugs to their toxic metabolites, is not expressed in latently-infected cells. Provided herein, in some non-claimed aspects, is a combination treatment wherein lytic replication is induced and antiviral drugs are administered concurrently.

For example, previous studies using patient-derived cells in vitro, and also from phase I/II clinical studies on a series of patients with EBV-associated lymphomas, have clearly shown the great promise of this combination therapy approach. Strong epidemiological association of Epstein-Barr Virus (EBV) with various human lymphoid malignancies and in vitro studies demonstrating tumorigenic activity of many EBV latent gene products suggest a causal relationship between EBV and these diseases. However, as EBV maintains a latent state of infection in these lymphomas, typical anti-herpesviral drugs, such as the nucleoside analogs ganciclovir (GCV) or acyclovir, are ineffective as these pro-drugs require expression of a lytic phase EBV protein, thymidine kinase (TK) or protein kinase (EBV-PK), for their activity. Therefore, selective induction of EBV lytic-phase gene expression in lymphoma cells that harbor latent EBV, coupled with simultaneous exposure to antiviral drugs, has been advanced as promising targeted therapy, because of resulting targeting of cytotoxicity to the EBV-infected tumor cells.

A variety of agents including short-chain fatty acids and chemotherapeutic drugs, have been used to induce EBV lytic-phase infection in cultured cells, but these in vitro studies have generally not resulted in clinical application. For instance, arginine butyrate and GCV has been used to treat EBV-positive lymphoid malignancies in a recent Phase I/II clinical trial. In this study of 15 patients with relapsed or refractory EBV-positive lymphoid tumors, 4 patients achieved complete tumor remissions and 6 patients partial tumor remissions. However, the rapid metabolism of butyrate requires continuous IV administration of high doses. Butyrate has pan-HDAC inhibitory activity, and it has been established that this activity is responsible for the induction of the EBV-TK protein. HDAC inhibitors may induce both EBV-TK and EBV-PK in EBV infected tumors. HDAC inhibitors may increase the activity of the CMV promoter in tumor cells. HDAC inhibitors may increase transcription of latent Herpes simplex virus genes in cell culture and tumors. In recent years, several potent HDAC inhibitors (HDACi) have been tested in the clinic as anti-cancer agents. In some instances, HDAC inhibitors induce lytic phase gene expression in viruses and kill virus-infected cells in combination with antiviral drugs. In certain instances, HDAC inhibitors, including some new, highly-potent compounds, induce EBV lytic phase gene expression and kill EBV-infected cells in combination with antiviral drugs. In some instances, HDAC inhibitors induce lytic phase gene expression in herpesviruses and kill virus-infected cells in combination with antiviral drugs.

### METHODS AND COMPOSITIONS

In one aspect, provided herein are methods for treating and/or preventing a condition in accordance with the claims wherein said antiviral agent is a nucleoside analog and said inducing agent is chidamide. The condition is caused by a latent viral infection. In certain embodiments, the condition is cancer, autoimmune or inflammatory condition. In certain embodiments, the cancer, autoimmune or inflammatory condition is caused by infection by Epstein-Barr Virus. In certain embodiments, the cancer, autoimmune or inflammatory condition is caused by infection by a Herpes simplex virus. In certain embodiments, the cancer, autoimmune or inflammatory condition is caused by infection by a cytomegalovirus. In certain embodiments, the methods comprise administering said viral inducing agent (an HDAC inhibitor which is chidamide according to the invention) and said antiviral agent. In some embodiments, the methods comprise administering said HDAC inhibitor and said antiviral agent. In certain embodiments, said HDAC inhibitor and said antiviral agent are co-formulated. In some embodiments, the methods comprise further administering an additional viral inducing agent. In other embodiments, the methods comprise further administering an additional antiviral agent. In some embodiments, the methods comprise administering additional individual doses of the viral inducing agent and/or the antiviral agent.

Further provided but not claimed herein are methods for treating and/or preventing a viral condition, a virally-induced condition, or an inflammatory condition comprising administering an HDAC inhibitor. The HDAC inhibitor may be a benzamide. According to the invention, the benzamide is chidamide. In a non-claimed aspect, the benzamide may be 4SC-202. The methods disclosed herein may further comprise administering an antiviral agent. The HDAC inhibitor and the antiviral agent may be co-formulated.

Also provided herein are methods for treating and/or preventing cancers in accordance with the claims. In some embodiments, the methods comprise administering an HDAC inhibitor and an antiviral wherein the HDAC inhibitor is a benzamide and wherein the benzamide is chidamide. In some embodiments, the antiviral is acyclovir and/or ganciclovir and/or valganciclovir.

Also provided but not claimed herein are compositions comprising an HDAC inhibitor and an antiviral agent. The HDAC inhibitor may be a benzamide. The benzamide HDAC inhibitor may be 4SC-202 or chidamide. The antiviral agent may be acyclovir, ganciclovir, or valganciclovir. The composition may comprise an additional agent. The additional agent may be an antiviral agent, an HDAC inhibitor, or a chemotherapeutic agent. The compositions may be formulated as a capsule, gel, tablet, solution, or suspension. The compositions may be formulated for oral administration. The compositions may be formulated for parenteral administration. The compositions may be formulated for intravenous, intraperitoneal, oral, subcutaneous, intrathecal, or intratumor administration. The compositions may be formulated for administration at the site of a viral infection. The compositions may be formulated for modified release of the HDAC inhibitor and the antiviral agent. The HDAC inhibitor may be dissolved before the antiviral agent is dissolved. The HDAC inhibitor may be dissolved after the antiviral agent is dissolved. The compositions may be formulated for once daily administration. The compositions may be formulated for twice daily, thrice daily, four times daily, once every other day, once weekly, once bi-weekly, or monthly.

The compositions may be administered in a cyclic or pulsatile manner. this may allow for a "drug holiday" or a "structured treatment interruption," which allows for the management of negative side-effects. The inducing agent and anti-viral agent may be administered for at least one cycle of therapy, said cycle comprising: (i) administering the inducing agent and the anti-viral agent to the subject over a first period of time; and (ii) continuing the administration of the anti-viral agent to the subject for a second period; wherein said second period represents the remainder of the cycle. The first period anti-viral agent and inducing agent may be administered in the same composition. The first period of time may be less than or equal to one-half of the length of the cycle. The first period of time may be less than or equal to about 5 days, and wherein said cycle is less than or equal to about 21 days.

### VIRAL INDUCING AGENTS

The methods of the provided invention comprise use of one or more pharmaceutical compositions provided herein comprising an inducing agent as defined in the claims to induce expression of a gene product in a virus-infected cell. The gene product expressed can be a viral enzyme or a cellular enzyme or activity that is largely expressed in virus-infected cells. Expression products that can be targeted include enzymes involved with DNA replication, for example, for repair or replication of the genome, assembly of complete virus particles, generation of viral membrane or walls, RNA transcription or protein translation or combinations of these activities. Interference with these processes can be performed by inducing and then acting on an enzyme and, preferably, a critical enzyme in the process.

According to the invention, the viral inducing agent is chidamide. According to the invention, the HDAC inhibitor is a benzamide, which is chidamide.

In certain non-claimed aspects, the molecular weight of the HDAC inhibitor is greater than 275 g/mol. In some embodiments, the HDAC inhibitor has a molecular weight of greater than 200 g/mol, greater than 225 g/mol, greater than 250 g/mol, greater than 275 g/mol, greater than 300 g/mol, greater than 325 g/mol, greater than 350 g/mol, greater than 375 g/mol, greater than 400 g/mol, greater than 425 g/mol, greater than 450 g/mol, greater than 475 g/mol, greater than 500 g/mol, greater than 525 g/mol, greater than 550 g/mol, greater than 575 g/mol, greater than 600 g/mol, greater than 625 g/mol, greater than 650 g/mol, greater than 675 g/mol, greater than 700 g/mol, greater than 725 g/mol, greater than 750 g/mol, greater than 775 g/mol, greater than 800 g/mol, greater than 850 g/mol, greater than 900 g/mol, greater than 950 g/mol, or greater than 1000 g/mol. In certain embodiments, the HDAC inhibitor has a molecular weight of less than 200 g/mol, less than 225 g/mol, less than 250 g/mol, less than 275 g/mol, less than 300 g/mol, less than 325 g/mol, less than 350 g/mol, less than 375 g/mol, less than 400 g/mol, less than 425 g/mol, less than 450 g/mol, less than 475 g/mol, less than 500 g/mol, less than 525 g/mol, less than 550 g/mol, less than 575 g/mol, less than 600 g/mol, less than 625 g/mol, less than 650 g/mol, less than 675 g/mol, less than 700 g/mol, less than 725 g/mol, less than 750 g/mol, less than 775 g/mol, less than 800 g/mol, less than 850 g/mol, less than 900 g/mol, less than 950 g/mol, or less than 1000 g/mol. In some embodiments, the HDAC inhibitor has a molecular weight of less than 500 g/mol and more than 250 g/mol. In other embodiments, the HDAC inhibitor has a molecular weight of less than 400 g/mol and more than 300 g/mol.

In a particular non-claimed aspect, the HDAC inhibitor is not m-carboxycinnamic acid, bishydroxamic acid, suberic bishydroxamic acid, Trichostatin A (7-[4-(dimethylamino)phenyl]-N-hydroxy-4,6-dimethyl-7-oxohepta-2,4-dienamide), SAHA (suberoyl anilide hydroxamic acid)/Vorinostat, oxamflatin, ABHA, SB-55629, SB939, pyroxamide, propenamides, aroyl pyrrolyl hydroxamides, Belinostat/PXD101, Papobinostat, LAQ824 (((E)-N-hydroxy-3-[4-[[2-hydroxyethyl-[2-(1H-indol-3-yl)ethyl]amino]methyl]phenyl]prop-2-enamide), LBH589, CI-994, Entinostat/MS-275, SNDX-275, or mocetinostat/ MGCD0103 (N-(2-aminophenyl)-4-((4-pyridin-3-ylpyrimidin-2-ylamino)methyl)benzamide).

In some embodiments, a viral inducing agent, for example an HDAC inhibitor, penetrates the blood brain barrier. In certain embodiments, a viral inducing agent that penetrates the blood brain barrier is a benzamide HDAC inhibitor. According to the invention, the viral inducing agent that penetrates the blood brain barrier comprises chidamide .

In some embodiments, the HDAC inhibitor is chidamide. In certain embodiments, chidamide is administered at a dose of 40 mg/day. In some embodiments, chidamide is administered at a dose of about 1 mg/day, about 2 mg/day, about 5 mg/day, about 10 mg/day, about 15 mg/day, about 20 mg/day, about 25 mg/day, about 30 mg/day, about 35 mg/day, about 40 mg/day, about 45 mg/day, about 50 mg/day, about 60 mg/day, about 70 mg/day, about 80 mg/day, about 90 mg/day, about 100 mg/day, about 150 mg/day, about 200 mg/day, about 250 mg/day, about 300 mg/day, about 350 mg/day, and about 400 mg/day. In certain embodiments, chidamide is administered at a dose of less than 1 mg/day, less than 2 mg/day, less than 5 mg/day, less than 10 mg/day, less than 15 mg/day, less than 20 mg/day, less than 25 mg/day, less than 30 mg/day, less than 35 mg/day, less than 40 mg/day, less than 45 mg/day, less than 50 mg/day, less than 60 mg/day, less than 70 mg/day, less than 80 mg/day, less than 90 mg/day, less than 100 mg/day, less than 150 mg/day, less than 200 mg/day, less than 250 mg/day, less than 300 mg/day, less than 350 mg/day, less than 400 mg/day. In some embodiments, chidamide is administered at a dose of more than 1 mg/day, more than 2 mg/day, more than 5 mg/day, more than 10 mg/day, more than 15 mg/day, more than 20 mg/day, more than 25 mg/day, more than 30 mg/day, more than 35 mg/day, more than 40 mg/day, more than 45 mg/day, more than 50 mg/day, more than 60 mg/day, more than 70 mg/day, more than 80 mg/day, more than 90 mg/day, more than 100 mg/day, more than 150 mg/day, more than 200 mg/day, more than 250 mg/day, more than 300 mg/day, more than 350 mg/day, or more than 400 mg/day. In some embodiments, chidamide is administered at a dose of about 1 mg/day to about 100 mg/day. In certain embodiments, chidamide is administered at a dose of about 5 mg/day to about 80 mg/day. In some embodiments, chidamide is administered at a dose of 5 mg twice weekly to 80 mg twice weekly. In some embodiments, chidamide is administered at a dose of 5 mg thrice weekly to 80 mg thrice weekly. In some embodiments, chidamide is administered at a dose of about 10 mg/day to about 60 mg/day. In some embodiments, chidamide is administered at a dose of 10 mg twice weekly to 60 mg twice weekly. In some embodiments, chidamide is administered at a dose of 10 mg thrice weekly to 60 mg thrice weekly. In some embodiments, chidamide is administered at a dose of 20 mg/day to 50 mg/day. In some embodiments, chidamide is administered at a dose of 20 mg twice weekly to 50 mg twice weekly. In some embodiments, chidamide is administered at a dose of 20 mg thrice weekly to 50 mg thrice weekly. In some embodiments, chidamide is administered at a dose of 30 mg/day to 40 mg/day. In some embodiments, chidamide is administered at a dose of 30 mg twice weekly to 40 mg twice weekly. In some embodiments, chidamide is administered at a dose of 30 mg thrice weekly to 40 mg thrice weekly. In certain embodiments, chidamide is administered once a day (q.d.), twice a day (b.id.), or thrice a day (t.i.d.). In some embodiments, chidamide is administered daily, once a week, twice a week, three times a week, four times a week, or five times a week. In some embodiments, chidamide is administered once a month, twice a month, thrice a month or 4 times a month. In certain embodiments, chidamide is in a delayed, slow or timed release form.

In some embodiments, a unit dose of a co-formulated HDAC inhibitor chidamide and antiviral agent comprises less than 400 mg of the HDAC inhibitor and less than 1000 mg of the antiviral agent. In some embodiments, a unit dose of a co-formulated HDAC inhibitor chidamide and antiviral agent comprises less than 200 mg of the HDAC inhibitor and less than 1000 mg of the antiviral agent. In some embodiments, a unit dose of a co-formulated HDAC inhibitor chidamide and antiviral agent comprises less than 100 mg of the HDAC inhibitor and less than 1000 mg of the antiviral agent. In certain embodiments, the unit dose comprises less than 50 mg of the HDAC inhibitor chidamide and less than 500 mg of the antiviral agent. In other embodiments, the unit dose comprises less than 80 mg of the HDAC inhibitor chidamide and less than 1500 mg of the antiviral agent. In some embodiments, the unit dose comprises less than 50 mg of the HDAC inhibitor chidamide and less than 1000 mg of the antiviral agent. In some embodiments, the unit dose comprises about 20 mg of the HDAC inhibitor chidamide and about 450 mg of the antiviral agent. In certain embodiments, the unit dose comprises about 40 mg of the HDAC inhibitor chidamide and about 900 mg of the antiviral agent. In some embodiments, the antiviral agent is formulated as controlled release.

The HDAC inhibitor disclosed but not claimed herein may be 4SC-202. 4SC-202 may be administered at a dose of 200 mg/day. 4SC-202 may be administered at a dose of about 1 mg/day, about 2 mg/day, about 5 mg/day, about 10 mg/day, about 15 mg/day, about 20 mg/day, about 25 mg/day, about 30 mg/day, about 35 mg/day, about 40 mg/day, about 45 mg/day, about 50 mg/day, about 60 mg/day, about 70 mg/day, about 80 mg/day, about 90 mg/day, about 100 mg/day, about 150 mg/day, about 200 mg/day, about 250 mg/day, about 300 mg/day, about 350 mg/day about 400 mg/day, about 450 mg/day or about 500 mg/day. 4SC-202 may be administered at a dose of less than 1 mg/day, less than 2 mg/day, less than 5 mg/day, less than 10 mg/day, less than 15 mg/day, less than 20 mg/day, less than 25 mg/day, less than 30 mg/day, less than 35 mg/day, less than 40 mg/day, less than 45 mg/day, less than 50 mg/day, less than 60 mg/day, less than 70 mg/day, less than 80 mg/day, less than 90 mg/day, or less than 100 mg/day, less than 150 mg/day, less than 200 mg/day, less than 250 mg/day, less than 300 mg/day, less than 350 mg/day less than 400 mg/day, less than 450 mg/day or less than 500 mg/day. 4SC-202 may be administered at a dose of more than 1 mg/day, more than 2 mg/day, more than 5 mg/day, more than 10 mg/day, more than 15 mg/day, more than 20 mg/day, more than 25 mg/day, more than 30 mg/day, more than 35 mg/day, more than 40 mg/day, more than 45 mg/day, more than 50 mg/day, more than 60 mg/day, more than 70 mg/day, more than 80 mg/day, more than 90 mg/day, more than 100 mg/day, more than 150 mg/day, more than 200 mg/day, more than 250 mg/day, more than 300 mg/day, more than 350 mg/day more than 400 mg/day, more than 450 mg/day or more than 500 mg/day. 4SC-202 may be administered at a dose of about 10 mg/day to about 1000 mg/day. 4SC-202 may be administered at a dose of about 20 mg/day to about 800 mg/day. 4SC-202 may be administered at a dose of about 25 mg/day to about 600 mg/day. 4SC-202 may be administered once a day (q.d.), twice a day (b.id.), or thrice a day (t.i.d.). The dose may be about 50 mg twice daily. The dose may be about 100 mg twice daily. The dose may be about 150 mg twice daily. The dose may be about 200 mg twice daily. The dose may be about 250 mg twice daily. The dose may be about 300 mg twice daily. 4SC-202 may be administered daily, once a week, twice a week, three times a week, four times a week, or five times a week. 4SC-202 may be administered once a month, twice a month, thrice a month or 4 times a month. 4SC-202 may be in a delayed, slow or timed release form.

A unit dose of a co-formulated HDAC inhibitor 4SC-202 and antiviral agent may comprise less than 400 mg of the HDAC inhibitor 4SC-202 and less than 1000 mg of the antiviral agent. A unit dose of a co-formulated HDAC inhibitor 4SC-202 and antiviral agent may comprise less than 200 mg of the HDAC inhibitor 4SC-202 and less than 1000 mg of the antiviral agent. The unit dose may comprise less than 50 mg of the HDAC inhibitor 4SC-202 and less than 500 mg of the antiviral agent. The unit dose may comprise less than 80 mg of the HDAC inhibitor 4SC-202 and less than 1500 mg of the antiviral agent. The unit dose may comprise less than 50 mg of the HDAC inhibitor 4SC-202 and less than 1000 mg of antiviral agent. The unit dose may comprise about 20 mg of the HDAC inhibitor 4SC-202 and about 450 mg of antiviral agent. The unit dose may comprise about 40 mg of the HDAC inhibitor 4SC-202 and about 900 mg of antiviral agent. The antiviral agent may be formulated as controlled release, delayed release or slow release.

The HDAC inhibitor disclosed but not claimed herein may be ACY-957 ACY-957 may be administered at a dose of 200 mg/day. ACY-957 may be administered at a dose of about 1 mg/day, about 2 mg/day, about 5 mg/day, about 10 mg/day, about 15 mg/day, about 20 mg/day, about 25 mg/day, about 30 mg/day, about 35 mg/day, about 40 mg/day, about 45 mg/day, about 50 mg/day, about 60 mg/day, about 70 mg/day, about 80 mg/day, about 90 mg/day, about 100 mg/day, about 150 mg/day, about 200 mg/day, about 250 mg/day, about 300 mg/day, about 350 mg/day about 400 mg/day, about 450 mg/day or about 500 mg/day. 4SC-202 may be administered at a dose of less than 1 mg/day, less than 2 mg/day, less than 5 mg/day, less than 10 mg/day, less than 15 mg/day, less than 20 mg/day, less than 25 mg/day, less than 30 mg/day, less than 35 mg/day, less than 40 mg/day, less than 45 mg/day, less than 50 mg/day, less than 60 mg/day, less than 70 mg/day, less than 80 mg/day, less than 90 mg/day, or less than 100 mg/day, less than 150 mg/day, less than 200 mg/day, less than 250 mg/day, less than 300 mg/day, less than 350 mg/day less than 400 mg/day, less than 450 mg/day or less than 500 mg/day. ACY-957 may be administered at a dose of more than 1 mg/day, more than 2 mg/day, more than 5 mg/day, more than 10 mg/day, more than 15 mg/day, more than 20 mg/day, more than 25 mg/day, more than 30 mg/day, more than 35 mg/day, more than 40 mg/day, more than 45 mg/day, more than 50 mg/day, more than 60 mg/day, more than 70 mg/day, more than 80 mg/day, more than 90 mg/day, more than 100 mg/day, more than 150 mg/day, more than 200 mg/day, more than 250 mg/day, more than 300 mg/day, more than 350 mg/day more than 400 mg/day, more than 450 mg/day or more than 500 mg/day. ACY-957 may be administered at a dose of about 10 mg/day to about 1000 mg/dayACY-957 may be administered at a dose of about 20 mg/day to about 800 mg/day. ACY-957 may be administered at a dose of about 25 mg/day to about 600 mg/day. ACY-957 may be administered once a day (q.d.), twice a day (b.id.), or thrice a day (t.i.d.). the dose may be about 50 mg twice daily. The dose may be about 100 mg twice daily. The dose may be about 150 mg twice daily. The dose may be about 200 mg twice daily. The dose may be about 250 mg twice daily. The dose may be about 300 mg twice daily. ACY-957 may be administered daily, once a week, twice a week, three times a week, four times a week, or five times a week. ACY-957 may be administered once a month, twice a month, thrice a month or 4 times a month. ACY-957 may be in a delayed, slow or timed release form.

A unit dose of a co-formulated HDAC inhibitor ACY-957 and antiviral agent may comprise less than 400 mg of the HDAC inhibitor ACY-957 and less than 1000 mg of the antiviral agent. A unit dose of a co-formulated HDAC inhibitor ACY-957and antiviral agent may comprise less than 200 mg of the HDAC inhibitor ACY-957 and less than 1000 mg of the antiviral agent. The unit dose may comprise less than 50 mg of the HDAC inhibitor ACY-957 and less than 500 mg of the antiviral agent. The unit dose may comprise less than 80 mg of the HDAC inhibitor ACY-957 and less than 1500 mg of the antiviral agent. The unit dose may comprise less than 50 mg of the HDAC inhibitor ACY-957 and less than 1000 mg of antiviral agent. The unit dose may comprise about 20 mg of the HDAC inhibitor ACY-957 and about 450 mg of antiviral agent. The unit dose may comprise about 40 mg of the HDAC inhibitor ACY-957 and about 900 mg of antiviral agent. The antiviral agent may be formulated as controlled release, delayed release or slow release.

The HDAC inhibitor disclosed but not claimed herein may be ACY-1215. ACY-1215 may be administered at a dose of 200 mg/day. ACY-1215 may be administered at a dose of about 1 mg/day, about 2 mg/day, about 5 mg/day, about 10 mg/day, about 15 mg/day, about 20 mg/day, about 25 mg/day, about 30 mg/day, about 35 mg/day, about 40 mg/day, about 45 mg/day, about 50 mg/day, about 60 mg/day, about 70 mg/day, about 80 mg/day, about 90 mg/day, about 100 mg/day, about 150 mg/day, about 160 mg/day, about 170 mg/day, about 200 mg/day, about 250 mg/day, about 300 mg/day, about 350 mg/day about 400 mg/day, about 450 mg/day or about 500 mg/day. 4SC-202 may be administered at a dose of less than 1 mg/day, less than 2 mg/day, less than 5 mg/day, less than 10 mg/day, less than 15 mg/day, less than 20 mg/day, less than 25 mg/day, less than 30 mg/day, less than 35 mg/day, less than 40 mg/day, less than 45 mg/day, less than 50 mg/day, less than 60 mg/day, less than 70 mg/day, less than 80 mg/day, less than 90 mg/day, or less than 100 mg/day, less than 150 mg/day, less than 200 mg/day, less than 250 mg/day, less than 300 mg/day, less than 350 mg/day less than 400 mg/day, less than 450 mg/day or less than 500 mg/day. ACY-1215 may be administered at a dose of more than 1 mg/day, more than 2 mg/day, more than 5 mg/day, more than 10 mg/day, more than 15 mg/day, more than 20 mg/day, more than 25 mg/day, more than 30 mg/day, more than 35 mg/day, more than 40 mg/day, more than 45 mg/day, more than 50 mg/day, more than 60 mg/day, more than 70 mg/day, more than 80 mg/day, more than 90 mg/day, more than 100 mg/day, more than 150 mg/day, more than 200 mg/day, more than 250 mg/day, more than 300 mg/day, more than 350 mg/day more than 400 mg/day, more than 450 mg/day or more than 500 mg/day. ACY-1215 may be administered at a dose of about 10 mg/day to about 1000 mg/day. ACY-1215 may be administered at a dose of about 20 mg/day to about 800 mg/day. ACY-1215 may be administered at a dose of about 25 mg/day to about 600 mg/day. ACY-1215 may be administered at a dose of about 50 mg/day to about 400 mg/day. ACY-1215 may be administered at a dose of about 100 mg/day to about 300 mg/day. ACY-1215 may be administered at a dose of about 100 mg/day to about 200 mg/day. ACY-1215 may be administered once a day (q.d.), twice a day (b.id.), or thrice a day (t.i.d.). The dose may be about 50 mg twice daily. The dose may be about 100 mg twice daily. The dose may be about 150 mg twice daily. The dose may be about 200 mg twice daily. The dose may be about 250 mg twice daily. The dose may be about 300 mg twice daily. ACY-1215 may be administered daily, once a week, twice a week, three times a week, four times a week, or five times a week. ACY-1215 may be administered once a month, twice a month, thrice a month or 4 times a month. ACY-1215 may be in a delayed, slow or timed release form.

A unit dose of a co-formulated HDAC inhibitor ACY-1215 and antiviral agent may comprise less than 400 mg of the HDAC inhibitor ACY-1215 and less than 1000 mg of the antiviral agent. A unit dose of a co-formulated HDAC inhibitor ACY-1215 and antiviral agent may comprise less than 200 mg of the HDAC inhibitor ACY-1215 and less than 1000 mg of the antiviral agent. The unit dose may comprise less than 50 mg of the HDAC inhibitor ACY-1215 and less than 500 mg of the antiviral agent. The unit dose may comprise less than 80 mg of the HDAC inhibitor ACY-1215 and less than 1500 mg of the antiviral agent. The unit dose may comprise less than 50 mg of the HDAC inhibitor ACY-1215 and less than 1000 mg of antiviral agent. The unit dose may comprise about 20 mg of the HDAC inhibitor ACY-1215 and about 450 mg of antiviral agent. The unit dose may comprise about 40 mg of the HDAC inhibitor ACY-1215 and about 900 mg of antiviral agent. IThe antiviral agent may be formulated as controlled release, delayed release or slow release.

In some embodiments, an HDAC inhibitor induces transcription of viral genes. In certain embodiments, the HDAC inhibitor induces transcription of EBV genes in EBV-positive lymphoma cells. In certain embodiments, the HDAC inhibitor induces transcription of CMV genes in CMV-positive cells. In certain embodiments, the HDAC induces transcription of herpesvirus genes in Herpes simplex virus infected cells. In some embodiments, the HDAC inhibitor induces transcription of viral genes at a concentration of about 100 µM, about 90 µM, about 80 µM, about 75 µM, about 70 µM, about 60 µM, about 50 µM, about 40 µM, about 30 µM, about 25 µM, about 20 µM, about 10 µM, about 5 µM, about 2 µM, about 1 µM, about 900 nM, about 800 nM, about 700 nM, about 600 nM, about 500 nM, about 400 nM, about 300 nM, about 200 nM, about 100 nM, about 75 nM, about 50 nM, about 20 nM, or about 10 nM. In certain embodiments, the HDAC inhibitor induces transcription of viral genes at a concentration of less than 100 µM, less than 90 µM, less than 80 µM, less than 75 µM, less than 70 µM, less than 60 µM, less than 50 µM, less than 40 µM, less than 30 µM, less than 25 µM, less than 20 µM, less than 10 µM, less than 5 µM, less than 2 µM, less than 1 µM, less than 900 nM, less than 800 nM, less than 700 nM, less than 600 nM, less than 500 nM, less than 400 nM, less than 300 nM, less than 200 nM, less than 100 nM, less than 75 nM, less than 50 nM, less than 20 nM, or less than 10 nM. In some embodiments, the HDAC inhibitor induces transcription of viral genes at a concentration of more than 100 µM, more than 90 µM, more than 80 µM, more than 75 µM, more than 70 µM, more than 60 µM, more than 50 µM, more than 40 µM, more than 30 µM, more than 25 µM, more than 20 µM, more than 10 µM, more than 5 µM, more than 2 µM, more than 1 µM, more than 900 nM, more than 800 nM, more than 700 nM, more than 600 nM, more than 500 nM, more than 400 nM, more than 300 nM, more than 200 nM, more than 100 nM, more than 75 nM, more than 50 nM, more than 20 nM, or more than 10 nM. In certain embodiments, the HDAC inhibitor induces transcription of viral genes at more than 50 nM and less than 100 nM. In some embodiments, the HDAC inhibitor has induces transcription of viral genes at more than 200 nM and less than 500 nM. In certain embodiments, the HDAC inhibitor induces transcription of viral genes at more than 100 nM and less than 200 nM.

### INDUCED GENES INCLUDING VIRAL-ASSOCIATED GENES

Inducing agents (agents that induce expression) may act directly on the viral genome or indirectly through a cellular factor required for viral expression. For example, viral gene expression can be regulated through the regulation of the expression of viral transcription factors such as ZTA, RTA, tat, and tax, cellular transcription factors such as AP-1, AP-2, Sp1, NF-κB, and other transcriptional activators and/or repressors (factors), co-activators and co- repressors, histone acetylators and deacetylators, DNA methylases and demethylases, oncogenes or proto-oncogenes, or protein kinase C. These proteins act to regulate and thereby control expression of specific viral and/or other cellular genetic elements. According to the methods of the invention, control over their expression can lead to control over the infection. Other gene products, both viral and cellular in origin, whose expression can be regulated with inducing agents include proteases, polymerases, reverse transcriptases, cell-surface receptors, major histocompatibility antigens, growth factors, and combination of these products.

Additional genes whose expression or transcriptional regulation are altered in the presence of butyric acid include the oncogenes myc, ras, myb, abl and src. The activities of these gene products, as well as the activities of other oncogenes, are described in Slamon, J.D., et al. 1984 Science 224:256-62. Anti-proliferative activity also includes the ability to repress tumor angiogenesis through the blockade of angiogenesis factor activity, production or release, transcriptional regulation, or the ability to modulate transcription of genes under angiogenesis or growth factor or hormonal control. Either would be an effective therapy, particularly against both prostatic neoplasia and breast carcinomas. Further activities that effect transcription and/or cellular differentiation include increased intracellular cAMP levels, inhibition of histone acetylation, and inhibition of genomic methylation. Each of these activities is directly related to gene expression, and increased expression can sensitize infected cells to a specific anti-viral agent.

In other non-claimed aspects, inducing agents include HDAC inhibitors that induce EBV-PK activity (also known BGLF4) in EBV infected tumors. Expression of EBV-PK/BGLF4 sensitizes a cell to an antiviral agent. In certain instances, HDAC inhibitors induce EBV-PK. In some instances, HDAC inhibitors induce EBV-TK and/or EBV-PK. In some instances, HDAC inhibitors induce HSV-TK and/or HSV-PK. In some instances, HDAC inhibitors induce CMV-TK and/or CMV-PK.

Preliminary *in vitro* studies according to the invention demonstrate that induction of EBV-TK activity in EBV-immortalized B-cells and patient-derived tumor cells using these drugs is possible, and that these previously resistant cells are rendered susceptible to ganciclovir therapy. Treatment of patients with viral-associated tumors such as EBV with inducing agents to induce the expression of EBV-TK/EBV-PK, and GCV, to eliminate EBV-TK/EBV-PK expressing tumor cells, is an effective, non-toxic therapy. This therapeutic regimen does not depend on the associated viral genome being the cause of the tumor. Without wishing to be bound by theory, it is believed that just the presence of the EBV genome in latent form would make the tumor susceptible to this combination protocol.

In some embodiments, an inducing agent induces viral gene expression by more than 4 fold after 24h of treatment. In certain embodiments, an HDAC inhibitor induces TK or EBV-PK expression by more than 4 fold after 24h of treatment. In some embodiments, an HDAC inhibitor induces viral gene expression after about 48h, about 36h, about 24h, about 18h, about 12h, about 8h, about 6h, about 4h, about 3h, about 2h, about 1h, or about 30 minutes. In certain embodiments, an HDAC inhibitor induces viral gene expression in less than 48h, less than 36h, less than 24h, less than 18h, less than 12h, less than 8h, less than 6h, less than 4h, less than 3h, less than 2h, less than 1h, or less than 30 minutes. In some embodiments, an HDAC inhibitor induces viral gene expression in more than 48h, more than 36h, more than 24h, more than 18h, more than 12h, more than 8h, more than 6h, more than 4h, more than 3h, more than 2h, more than 1h, or more than 30 minutes. In certain embodiments, an HDAC inhibitor induces viral gene expression after more than 30 minutes and less than 24h.

In certain embodiments, an inducing agent is capable of inducing gene expression at a concentration of less than 500 nM. In some embodiments, the inducing agent is an HDAC inhibitor According to the invention, the inducing agent is chidamide. In certain embodiments, the inducing agent is capable of inducing EBV-TK or EBV-PK expression. In certain embodiments, the inducing agent is capable of inducing HSV-TK or HSV-PK expression. In certain embodiments, the inducing agent is capable of inducing CMV-TK or CMV-PK expression. In certain embodiments, an inducing agent is capable of inducing gene expression at a concentration of about 100 µM, about 90 µM, about 80 µM, about 75 µM, about 70 µM, about 60 µM, about 50 µM, about 40 µM, about 30 µM, about 25 µM, about 20 µM, about 10 µM, about 5 µM, about 2 µM, about 1 µM, about 900 nM, about 800 nM, about 700 nM, about 600 nM, about 500 nM, about 400 nM, about 300 nM, about 200 nM, about 100 nM, about 75 nM, about 50 nM, about 20 nM, or about 10 nM. In some embodiments, an inducing agent is capable of inducing gene expression at a concentration of less than 100 µM, less than 90 µM, less than 80 µM, less than 75 µM, less than 70 µM, less than 60 µM, less than 50 µM, less than 40 µM, less than 30 µM, less than 25 µM, less than 20 µM, less than 10 µM, less than 5 µM, less than 2 µM, less than 1 µM, less than 900 nM, less than 800 nM, less than 700 nM, less than 600 nM, less than 500 nM, less than 400 nM, less than 300 nM, less than 200 nM, less than 100 nM, less than 75 nM, less than 50 nM, less than 20 nM, or less than 10 nM. In certain embodiments, an inducing agent is capable of inducing gene expression at a concentration of more than 100 µM, more than 90 µM, more than 80 µM, more than 75 µM, more than 70 µM, more than 60 µM, more than 50 µM, more than 40 µM, more than 30 µM, more than 25 µM, more than 20 µM, more than 10 µM, more than 5 µM, more than 2 µM, more than 1 µM, more than 900 nM, more than 800 nM, more than 700 nM, more than 600 nM, more than 500 nM, more than 400 nM, more than 300 nM, more than 200 nM, more than 100 nM, more than 75 nM, more than 50 nM, more than 20 nM, or more than 10 nM. In some embodiments, an inducing agent is capable of inducing gene expression at a concentration more than 50 nM and less than 100 nM. In certain embodiments, an inducing agent is capable of inducing gene expression at a concentration of more than 200 nM and less than 500 nM. In some embodiments, an inducing agent is capable of inducing gene expression at more than 100 nM and less than 200 nM

In some embodiments, an HDAC inhibitor induces viral gene expression after more than 1h and less than 6h. In certain embodiments, an HDAC inhibitor induces viral gene expression about 2 fold, about 3 fold, about 4 fold, about 5 fold, about 6 fold, about 7 fold, about 8 fold, about 9 fold, about 10 fold, about 12 fold, about 15 fold, about 20 fold, about 25 fold, about 30 fold, about 35 fold, about 40 fold, about 45 fold, or about 50 fold. In some embodiments, an HDAC inhibitor induces viral gene expression less than 2 fold, less than 3 fold, less than 4 fold, less than 5 fold, less than 6 fold, less than 7 fold, less than 8 fold, less than 9 fold, less than 10 fold, less than 12 fold, less than 15 fold, less than 20 fold, less than 25 fold, less than 30 fold, less than 35 fold, less than 40 fold, less than 45 fold, or less than 50 fold. In certain embodiments, an HDAC inhibitor induces viral gene expression more than 2 fold, more than 3 fold, more than 4 fold, more than 5 fold, more than 6 fold, more than 7 fold, more than 8 fold, more than 9 fold, more than 10 fold, more than 12 fold, more than 15 fold, more than 20 fold, more than 25 fold, more than 30 fold, more than 35 fold, more than 40 fold, more than 45 fold, or more than 50 fold. In some embodiments, an HDAC inhibitor induces viral gene expression more than 2 fold and less than 50 fold. In certain embodiments, an HDAC inhibitor induces viral gene expression more than 5 fold and less than 40 fold.

### ANTIVIRAL AGENTS

Anti-viral agents that can be used in the compositions disclosed but not claimed herein can include, for example, substrates and substrate analogs, inhibitors and other agents that severely impair, debilitate or otherwise destroy virus-infected cells. Substrate analogs include amino acid and nucleoside analogs. Substrates can be conjugated with toxins or other viricidal substances. Inhibitors include integrase inhibitors, protease inhibitors, polymerase inhibitors and transcriptase inhibitors such as reverse transcriptase inhibitors.

Antiviral agents that can be used in the compositions disclosed but not claimed herein can include, for example, ganciclovir, valganciclovir, oseltamivir (Tamiflu^{™}), zanamivir (Relenza^{™}), abacavir, aciclovir, acyclovir, adefovir, amantadine, amprenavir, ampligen, arbidol, atazanavir, atripla, boceprevir, cidofovir, combivir, darunavir, delavirdine, didanosine, docosanol, edoxudine, efavirenz, emtricitabine, enfuvirtide, entecavir, famciclovir, fomivirsen, fosamprenavir, foscarnet, fosfonet, fusion inhibitors (e.g., enfuvirtide), ibacitabine, imunovir, idoxuridine, imiquimod, indinavir, inosine, integrase inhibitor, interferon type III, interferon type II, interferon type I, interferon, lamivudine, lopinavir, loviride, maraviroc, moroxydine, nelfinavir, nevirapine, nexavir, nucleoside analogues, peginterferon alfa-2a, penciclovir, peramivir, pleconaril, podophyllotoxin, protease inhibitor, raltegravir, reverse transcriptase inhibitor, ribavirin, rimantadine, ritonavir, pyrimidine antiviral, saquinavir, stavudine, synergistic enhancer (antiretroviral), tenofovir, tenofovir disoproxil, tipranavir, trifluridine, trizivir, tromantadine, truvada, valaciclovir (Valtrex^{™}), vicriviroc, vidarabine, viramidine, zalcitabine, and zidovudine.

In a specific embodiment according to the invention, the antiviral agent is acyclovir, ganciclovir, or valganciclovir.

According to the invention, the antiviral agent is a nucleoside analog. Examples of nucleoside analogs include acyclovir (ACV), ganciclovir (GCV), valganciclovir, famciclovir, ribavirin, zalcitabine (ddC), zidovudine (AZT), stavudine (D4T), lamivudine (3TC), didanosine (ddI), cytarabine, dideoxyadenosine, edoxudine, floxuridine, idoxuridine, inosine pranobex, 2'-deoxy-5-(methylamino)uridine, trifluridine and vidarabine. Examples of a few protease inhibitors that show particular promise in human therapy include saquinivir, ritonavir and indinavir. Other anti-viral agents include interferons (e.g. α-, β-, γ-interferon), cytokines such as tumor necrosis factor (TNF) or interleukins, cell receptors and growth factor antagonists, which can be purified or recombinantly produced.

In some embodiments, the antiviral agent is administered at a dose of less than 3000 mg/day. In some embodiments, the antiviral agent is administered at a dose of about 10 mg/day, about 20 mg/day, about 50 mg/day, about 100 mg/day, about 150 mg/day, about 200 mg/day, about 250 mg/day, about 300 mg/day, about 350 mg/day, about 400 mg/day, about 450 mg/day, about 500 mg/day, about 600 mg/day, about 700 mg/day, about 800 mg/day, about 900 mg/day, about 1000 mg/day, about 1200 mg/day, about 1250 mg/day, about 1400 mg/day, about 1500 mg/day, about 1600 mg/day, about 1750 mg/day, about 1800 mg/day, about 1900 mg/day, about 2000 mg/day, about 2250 mg/day, about 2500 mg/day, about 2750 mg/day, about 3000 mg/day, about 3250 mg/day, about 350 0mg/day, about 3750 mg/day, about 4000 mg/day, about 4250 mg/day, about 4500 mg/day, about 4750 mg/day, or about 5000 mg/day. In certain embodiments, the antiviral agent is administered at a dose of less than 10 mg/day, less than 20 mg/day, less than 50 mg/day, less than 100 mg/day, less than 150 mg/day, less than 200 mg/day, less than 250 mg/day, less than 300 mg/day, less than 350 mg/day, less than 400 mg/day, less than 450 mg/day, less than 500 mg/day, less than 600 mg/day, less than 700 mg/day, less than 800 mg/day, less than 900 mg/day, less than 1000 mg/day, less than 1200 mg/day, less than 1250 mg/day, less than 1400 mg/day, less than 1500 mg/day, less than 1600 mg/day, less than 1750 mg/day, less than 1800 mg/day, less than 1900 mg/day, less than 2000 mg/day, less than 2250 mg/day, less than 2500 mg/day, less than 2750 mg/day, less than 3000 mg/day, less than 3250 mg/day, less than 3500 mg/day, less than 3750 mg/day, less than 4000 mg/day, less than 4250 mg/day, less than 4500 mg/day, less than 4750 mg/day, or less than 5000 mg/day. In some embodiments, the antiviral agent is administered at a dose of more than 10 mg/day, more than 20 mg/day, more than 50 mg/day, more than 100 mg/day, more than 150 mg/day, more than 200 mg/day, more than 250 mg/day, more than 300 mg/day, more than 350 mg/day, more than 400 mg/day, more than 450 mg/day, more than 500 mg/day, more than 600 mg/day, more than 700 mg/day, more than 800 mg/day, more than 900 mg/day, more than 1000 mg/day, more than 1200 mg/day, more than 1250 mg/day, more than 1400 mg/day, more than 1500 mg/day, more than 1600 mg/day, more than 1750 mg/day, more than 1800 mg/day, more than 1900 mg/day, more than 2000 mg/day, more than 2250 mg/day, more than 2500 mg/day, more than 2750 mg/day, more than 3000 mg/day, more than 3250 mg/day, more than 3500 mg/day, more than 3750 mg/day, more than 4000 mg/day, more than 4250 mg/day, more than 4500 mg/day, more than 4750 mg/day, or more than 5000 mg/day. In certain embodiments, the antiviral agent is administered at a dose of more than 10 mg/day and less than 5000 mg/day. In some embodiments, the antiviral agent is administered at a dose of more than 200 mg/day and less than 1000 mg/day. In certain embodiments, the antiviral agent is administered once a day (q.d.), twice a day (b.id.), or thrice a day (t.i.d.). In some embodiments, the antiviral agent is administered daily, once a week, twice a week, three times a week, four times a week, or five times a week.

In certain embodiments, the antiviral agent is ganciclovir. In some embodiments, ganciclovir is administered at a total daily dose of 3000 mg/day. In certain embodiments, ganciclovir is administered at a dose of 1000 mg three times a day. In some embodiments, ganciclovir is administered at a dose of about 100 mg/day, about 250 mg/day, about 500 mg/day, about 750 mg/day, about 1000 mg/day, about 1500 mg/day, about 2000 mg/day, about 2500 mg/day, about 3000 mg/day, about 3500 mg/day, or about 4000 mg/day. In certain embodiments, ganciclovir is administered at a dose of less than 100 mg/day, less than 250 mg/day, less than 500 mg/day, less than 750 mg/day, less than 1000 mg/day, less than 1500 mg/day, less than 2000 mg/day, less than 2500 mg/day, less than 3000 mg/day, less than 3500 mg/day, or less than 4000 mg/day. In some embodiments, ganciclovir is administered at a dose of more than 100 mg/day, more than 250 mg/day, more than 500 mg/day, more than 750 mg/day, more than 1000 mg/day, more than 1500 mg/day, more than 2000 mg/day, more than 2500 mg/day, more than 3000 mg/day, more than 3500 mg/day, or more than 4000 mg/day. In certain embodiments, ganciclovir is administered at a dose of more than 500 mg/day and less 4000 mg/day. In some embodiments, ganciclovir is administered at a dose of more than 1000 mg/day and less than 3000 mg/day. In some embodiments, ganciclovir is administered once a day, twice a day, or three times a day. In certain embodiments, ganciclovir is administered once a week, twice a week, three times a week, four times a week, five times a week, or daily.

In some embodiments, the antiviral agent is valganciclovir. In certain embodiments, valganciclovir is administered at a total daily dose of 900 mg/day. In some embodiments, valganciclovir is administered at a dose of 900 mg once a day. In certain embodiments, valganciclovir is administered at a total daily dose of 1800 mg/day. In some embodiments, valganciclovir is administered at a dose of 900 mg twice a day.

In some embodiments, valganciclovir is administered at a dose of about 100 mg/day, about 200 mg/day, about 300 mg/day, about 400 mg/day, about 500 mg/day, about 600 mg/day, about 700 mg/day, about 800 mg/day, about 900 mg/day, about 1000 mg/day, about 1100 mg/day, about 1200 mg/day, about 1300 mg/day, about 1400 mg/day, about 1500 mg/day, about 1600 mg/day, about 1700 mg/day, about 1800 mg/day, about 1900 mg/day, or about 2000 mg/day. In certain embodiments, valganciclovir is administered at a dose of less than 100 mg/day, less than 200 mg/day, less than 300 mg/day, less than 400 mg/day, less than 500 mg/day, less than 600 mg/day, less than 700 mg/day, less than 800 mg/day, less than 900 mg/day, less than 1000 mg/day, less than 1100 mg/day, less than 1200 mg/day, less than 1300 mg/day, less than 1400 mg/day, less than 1500 mg/day, less than 1600 mg/day, less than 1700 mg/day, less than 1800 mg/day, less than 1900 mg/day, or less than 2000 mg/day. In some embodiments, valganciclovir is administered at a dose of more than 100 mg/day, more than 200 mg/day, more than 300 mg/day, more than 400 mg/day, more than 500 mg/day, more than 600 mg/day, more than 700 mg/day, more than 800 mg/day, more than 900 mg/day, more than 1000 mg/day, more than 1100 mg/day, more than 1200 mg/day, more than 1300 mg/day, more than 1400 mg/day, more than 1500 mg/day, more than 1600 mg/day, more than 1700 mg/day, more than 1800 mg/day, more than 1900 mg/day, or more than 2000 mg/day. In certain embodiments, valganciclovir is administered at a dose of more than 100 mg/day and less 2000 mg/day. In some embodiments, valganciclovir is administered at a dose of more than 500 mg/day and less than 1500 mg/day. In some embodiments, valganciclovir is administered once a day, twice a day, or three times a day. In certain embodiments, valganciclovir is administered once a week, twice a week, three time a week, four times a week, five times a week, or daily.

In a non-claimed aspect, the antiviral agent is not a heat shock protein inhibitor, an immunosuppressant, an antibiotic, a glucocorticoid, a non-steroidal anti-inflammatory drug, a Cox-2-specific inhibitor or a TNF-α binding protein. In a related embodiment, the antiviral agent is not a Hsp90 inhibitor, tacrolimus, cyclosporin, rapamycin (sirolimus), methotrexate, cyclophosphamide, azathioprine, mercaptopurine, mycophenolate, FTY720, levofloxacin, amoxycillin, prednisone, cortisone acetate, prednisolone, methylprednisolone, dexamethasone, betamethasone, triamcinolone, beclometasone, fludrocortisone acetate, deoxycorticosterone acetate, aldosterone, salicylates, arylalkanoic acids, a 2-arylpropionic acid, a N-arylanthranilic acid, an oxicam, a coxib, a sulphonanilide, valdecoxib, celecoxib, rofecoxib, leflunomide, gold thioglucose, gold thiomalate, aurofin, sulfasalazine, hydroxychloroquinine, minocycline, infliximab, etanercept, adalimumab, abatacept, anakinra, interferon-β, interferon-γ, interleukin-2, an allergy vaccine, an antihistamine, an antileukotriene, a beta-agonist, theophylline, or an anticholinergic.

### ADDITIONAL AGENTS

The methods of the provided invention can comprise administering to a subject a viral inducing agent, and antiviral agent, as defined in the claims, and one or more additional active agents. The additional agent can be selected based on the type of viral, virally-induced, or inflammatory condition the subject has or is suspected of having. The additional agent can comprise, for example, another antiviral agent, another viral inducing agent, a vaccine, or an anticancer agent. For example, a subject with multiple sclerosis can be administered a viral inducing agent, an antiviral agent, and a vaccine, for example, a vaccine comprising myelin basic protein. In another example, a subject with diabetes can be administered a viral inducing agent, an antiviral agent, and a vaccine, for example, a vaccine comprising an antigen. An additional agent disclosed herein may be an anticancer agent. The anticancer agent may be a chemotherapeutic anticancer agent. Examples of chemotherapeutic anticancer agents include, but are not limited to, nitrogen mustards; alkyl sulfonates; ethylene imines; nitrosoureas; epoxides; other alkylating agents; folic acid analogues; purine analogs; pyrimidine analogs; vinca alkaloids; podophyllotoxin derivatives; colchicine derivatives; taxanes; other plant alkaloids and natural products; actinomycines; antracyclines; other cytotoxic antibiotics; platinum compounds; methylhydrazines; sensitizers; protein kinase inhibitors; other antineoplastic agents; estrogens; progestogens; gonadotropin releasing hormone analogs; anti-estrogens; anti-androgens; enzyme inhibitors; other hormone antagonists; immunostimulants; immunosuppressants; calcineurin inhibitors; and radiopharmaceuticals. The anticancer agent may be a toxin, e.g. diphtheria toxin. The anticancer agent may be an antibody. The antibody may be monoclonal, polyclonal, chimeric, or humanized or conjugated to a radioisotope, toxin, or cytotoxic chemical. The anticancer antibody may be directed to CD20, CD22, CD30, CD40, CD52. The anticancer antibody may be rituximab, veltuzumab, ofatumumab, ocrelizumab, GA-101, epratuzumab, 90Y-epratuzumab, tetraxetan, inotuzumab ozogamicin, BL22, HA22, dacetuzumab, lucatumumab, SGN-30, brentuximab vedotin, blinatumomab, ibritunib, lenalidomide, pertuzumab, trastuzumab, mapatumumab, gemtuzumab ozogamicin, tositumomab, ibritumomab tiuxetan, bevacizumab, volociximab, etaracizumab, cetuximab, panitumumab, nimotuzumab, denosumab, sibrotuzumab, IGN101, adecatumumab, labetuzumab, pemtumomab, oregovomab, farletuzumab, CC49 (minretumomab), cG250, J591, IM-2C6, CDP791, hu3S193 and IgN311, KB004, IIIA4, and mapatumumab (HGS-ETR1).

An additional agent may be a non-steroidal anti-inflammatory agent (NSAID). NSAID include, for example, Aspirin (Anacin^{™}, Ascriptin^{™}, Bayer^{™}, Bufferin^{™}, Ecotrin^{™}, Excedrin^{™}), Choline and magnesium salicylates (CMT^{™}, Tricosal^{™}, Trilisate^{™}), choline salicylate (Arthropan^{™}), celecoxib (Celebrex^{™}), diclofenac potassium (Cataflam^{™}), diclofenac sodium (Voltaren^{™}, Voltaren XR^{™}), diclofenac sodium with misoprostol (Arthrotec^{™}), diflunisal (Dolobid^{™}), etodolac (Lodine^{™}, Lodine XL^{™}), fenoprofen calcium (Nalfon^{™}), flurbiprofen (Ansaid^{™}), ibuprofen (Advil^{™}, Motrin^{™}, Motrin IB^{™}, Nuprin^{™}), indomethacin (Indocin^{™}, Indocin SR^{™}), ketoprofen (Actron^{™}, Orudis^{™}, Orudis KT^{™}, Oruvail^{™}), magnesium salicylate (Arthritab^{™}, Bayer Select^{™}, Doan's Pills^{™}, Magan^{™}, Mobidin^{™}, Mobogesic^{™}), meclofenamate sodium (Meclomen^{™}), mefenamic acid (Ponstel^{™}), meloxicam (Mobic^{™}), nabumetone (Relafen^{™}), naproxen (Naprosyn^{™}, Naprelan^{™}), naproxen sodium (Aleve^{™}, Anaprox^{™}), oxaprozin (Daypro^{™}), piroxicam (Feldene^{™}), rofecoxib (Vioxx^{™}), salsalate (Amigesic^{™}, Anaflex 750^{™}, Disalcid^{™}, Marthritic^{™}, Mono-Gesic^{™}, Salflex^{™}, Salsitab^{™}), sodium salicylate (various generics), sulindac (Clinoril^{™}), tolmetin sodium (Tolectin^{™}), valdecoxib (Bextra^{™}).

An additional agent may be a lipid lowering agent. The lipid lowering agent may be a statin. Examples of statins include, but are not limited to, Advicor^{®} (niacin extended-release/lovastatin), Altoprev^{®} (lovastatin extended-release), Caduet^{®} (amlodipine and atorvastatin), Crestor^{®} (rosuvastatin), Lescol^{®} (fluvastatin), Lescol XL (fluvastatin extended-release), Lipitor^{®} (atorvastatin), Mevacor^{®} (lovastatin), Pravachol^{®} (pravastatin), Simcor^{®} (niacin extended-release/simvastatin), Vytorin^{®} (ezetimibe/simvastatin), and Zocor^{®} (simvastatin). A lipid lowering agent can be administered to a subject that has or is suspected of having atherosclerosis. For example, a subject with cytomegalovirus induced atherosclerosis can be administered an additional agent that can comprise atorvastatin, rosuvastatin, lovastatin, simvastatin, or pravastatin.

An additional agent disclosed herein may be an immunosuppressive drug. Immunosuppressive drug, for example, include glucocorticoids, antibodies, cytostatic agents, and drugs that act on immunophilins. Glucocorticoids can include, for example, prednisolone, prednisone, or methylprednisolone. A cytostatic agent can include, for example, an agent that interferes with nucleic acid synthesis, for example, folic acid, pyrimidine analogs, and purine analogs. A folic acid analog that can be used as an immunosuppressive drug is methotrexate, which can bind dihydrofolate reductase and prevent the synthesis of tetrahydrofolate. Another cytostatic agent is azathioprine, which can be cleaved non enzymatically to form mercaptopurine, which can act as a purine analogue. A cytostatic agent can include, for example, an alkylating agent, including, for example, cyclophosphamide, and a nitrosourea. A cytostatic agent can be a platinum compound. Other cytostatic agents include, for example, cytotoxic antibiotics, including dactinomycin, anthracylcines, mitomycin C, bleomycin, and mithramycin. Examples of antibodies that can be immunosuppressive agents include, for example, heterologous polyclonal antibodies, for example, from rabbit or horse. Other antibodies include monoclonal antibodies directed to specific antigens e.g., T-cell receptor directed antibodies (e.g., OKT3, muromonab, which targets CD3), and IL-2 receptor directed antibodies (e.g., targeting CD25). Drugs that can act on immunophilins include, for example, cyclosporin, tacrolimus (Prograf), Sirolimus (rapamycin, Rapamune). Other drugs that can act as immunosuppressive drugs include, for example, mycophenolate (mycophenolic acid), interferons, opioids, TNF binding proteins, Fingolimod, myriocin, and ciclosporin.

The additional agent can be, for example, FK506, a monoclonal antibody, an anti-T cell monoclonal antibody, an anti-B cell monoclonal antibody, or a TNF inhibitor. The monoclonal antibody can be an anti-B cell antibody. The anti-B cell antibody can be anti-CD20. The TNF inhibitor can be infliximab (Remicade^{™}), etanercept (Enbrel^{™}), adalimumab (Humira^{™}), or an anti-IL-6 antibody.

A subject with an autoimmune condition can be administered a viral inducing agent, an antiviral agent, and an additional agent, where the additional agent comprises cyclosporine, azathiorprine, methotrexate, cyclophosphamide, FK506, tacrolimus, a monoclonal antibody, an anti-T cell monoclonal antibody, an anti-B cell monoclonal antibody, an IL-2 receptor antibody, or a TNF inhibitor.

The additional agent can be glatiramer (Copaxone^{™}), Natalizumab (Tysabri^{™}), mitoxantrone (Novantrone^{™}), cladribine, or Campath antibody. For example, a subject with multiple sclerosis can be administered an additional agent that can comprise glatiramer, mitoxantrone, natalizumab, cladribine, or Campath antibody.

### TYPES OF VIRUSES AND VIRALLY-INDUCED CONDITIONS

The methods and compositions provided but not claimed herein can be used to treat and/or prevent viral infections. The virus causing the infection can be a member of the herpesvirus family, a human immunodeficiency virus, parvovirus, or coxsackie virus. According to the invention, the virus is a herpesvirus. A member of the herpesvirus family can be herpes simplex virus, herpes genitalis virus, varicella zoster virus, Epstein-Barr virus, human herpesvirus 6, human herpesvirus 8, or cytomegalovirus. The subject can have coronary artery condition associated with a cytomegalovirus or herpes simplex virus infection. The subject can have an autoimmune condition associated with Epstein-Barr virus infection. The subject can have a lymphoma or other cancer associated with Epstein-Barr virus infection. The subject can have a lymphoma or other cancer associated with human herpesvirus 8 infection. The subject can have an autoimmune condition associated with Herpes simplex virus infection. The subject can have a cancer associate with herpes simplex virus. The subject can have an autoimmune condition associated with cytomegalovirus infection. The subject can have a lymphoma or other cancer associated with cytomegalovirus infection.

In a non-claimed aspect, the viral or virally-induced condition may be caused by a retrovirus, such as HIV, HTLV1 and 2. The viral or virally-induced condition may be caused by a DNA virus, such as a herpesvirus (according to the invention). The herpesvirus may be an Epstein-Barr virus, cytomegalovirus, Herpes simplex type 1, herpes simplex type 2, Kaposi's sarcoma virus (human herpesvirus 8), BK viruses, or hepatitis virus. The virally-induced or virus-associated disease may be a cancer. The virally-induced or virus-associated cancer may be a lymphoma, chronic lymphocytic leukemia, NK/T cell lymphoma, primary effusion lymphoma, nasopharyngeal carcinoma, gastric cancer, or Kaposi's sarcoma. The virally-induced or virus-associated disease may be an autoimmune disease. The autoimmune disease may be rheumatoid arthritis, systemic lupus erythematosus, or multiple sclerosis.

The methods and compositions described but not claimed herein can be used to treat and/or prevent infections caused by any virus, including, for example, Abelson leukemia virus, Abelson murine leukemia virus, Abelson's virus, Acute laryngotracheobronchitis virus, Adelaide River virus, Adeno associated virus group, Adenovirus, African horse sickness virus, African swine fever virus, AIDS virus, Aleutian mink condition parvovirus, Alpharetrovirus, Alphavirus, ALV related virus, Amapari virus, Aphthovirus, Aquareovirus, Arbovirus, Arbovirus C, arbovirus group A, arbovirus group B, Arenavirus group, Argentine hemorrhagic fever virus, Argentine haemorrhagic fever virus, Arterivirus, Astrovirus, Ateline herpesvirus group, Aujezky's condition virus, Aura virus, Ausduk condition virus, Australian bat lyssavirus, Aviadenovirus, avian erythroblastosis virus, avian infectious bronchitis virus, avian leukemia virus, avian leukosis virus, avian lymphomatosis virus, avian myeloblastosis virus, avian paramyxovirus, avian pneumoencephalitis virus, avian reticuloendotheliosis virus, avian sarcoma virus, avian type C retrovirus group, Avihepadnavirus, Avipoxvirus, B virus, B19 virus, Babanki virus, baboon herpesvirus, baculovirus, Barmah Forest virus, Bebaru virus, Berrimah virus, Betaretrovirus, Birnavirus, Bittner virus, BK virus, Black Creek Canal virus, bluetongue virus, Bolivian hemorrhagic fever virus, Boma condition virus, border condition of sheep virus, borna virus, bovine alphaherpesvirus 1, bovine alphaherpesvirus 2, bovine coronavirus, bovine ephemeral fever virus, bovine immunodeficiency virus, bovine leukemia virus, bovine leukosis virus, bovine mammillitis virus, bovine papillomavirus, bovine papular stomatitis virus, bovine parvovirus, bovine syncytial virus, bovine type C oncovirus, bovine viral diarrhea virus, Buggy Creek virus, bullet shaped virus group, Bunyamwera virus supergroup, Bunyavirus, Burkitt's lymphoma virus, Bwamba Fever, CA virus, Calicivirus, California encephalitis virus, camelpox virus, canarypox virus, canid herpesvirus, canine coronavirus, canine distemper virus, canine herpesvirus, canine minute virus, canine parvovirus, Cano Delgadito virus, caprine arthritis virus, caprine encephalitis virus, Caprine Herpes Virus, Capripox virus, Cardiovirus, caviid herpesvirus 1, Cercopithecid herpesvirus 1, cercopithecine herpesvirus 1, Cercopithecine herpesvirus 2, Chandipura virus, Changuinola virus, channel catfish virus, Charleville virus, chickenpox virus, Chikungunya virus, chimpanzee herpesvirus, chub reovirus, chum salmon virus, Cocal virus, Coho salmon reovirus, coital exanthema virus, Colorado tick fever virus, Coltivirus, Columbia SK virus, common cold virus, contagious ecthyma virus, contagious pustular dermatitis virus, Coronavirus, Corriparta virus, coryza virus, cowpox virus, coxsackie virus, CPV (cytoplasmic polyhedrosis virus), cricket paralysis virus, Crimean-Congo hemorrhagic fever virus, croup associated virus, Cryptovirus, Cypovirus, Cytomegalovirus, cytomegalovirus group, cytoplasmic polyhedrosis virus, deer papillomavirus, deltaretrovirus, dengue virus, Densovirus, Dependovirus, Dhori virus, diplorna virus, Drosophila C virus, duck hepatitis B virus, duck hepatitis virus 1, duck hepatitis virus 2, duovirus, Duvenhage virus, Deformed wing virus DWV, eastern equine encephalitis virus, eastern equine encephalomyelitis virus, EB virus, Ebola virus, Ebola-like virus, echo virus, echovirus, echovirus 10, echovirus 28, echovirus 9, ectromelia virus, EEE virus, EIA virus, EIA virus, encephalitis virus, encephalomyocarditis group virus, encephalomyocarditis virus, Enterovirus, enzyme elevating virus, enzyme elevating virus (LDH), epidemic hemorrhagic fever virus, epizootic hemorrhagic condition virus, Epstein-Barr virus, equid alphaherpesvirus 1, equid alphaherpesvirus 4, equid herpesvirus 2, equine abortion virus, equine arteritis virus, equine encephalosis virus, equine infectious anemia virus, equine morbillivirus, equine rhinopneumonitis virus, equine rhinovirus, Eubenangu virus, European elk papillomavirus, European swine fever virus, Everglades virus, Eyach virus, felid herpesvirus 1, feline calicivirus, feline fibrosarcoma virus, feline herpesvirus, feline immunodeficiency virus, feline infectious peritonitis virus, feline leukemia /sarcoma virus, feline leukemia virus, feline panleukopenia virus, feline parvovirus, feline sarcoma virus, feline syncytial virus, Filovirus, Flanders virus, Flavivirus, foot and mouth condition virus, Fort Morgan virus, Four Corners hantavirus, fowl adenovirus 1, fowlpox virus, Friend virus, Gammaretrovirus, GB hepatitis virus, GB virus, German measles virus, Getah virus, gibbon ape leukemia virus, glandular fever virus, goatpox virus, golden shinner virus, Gonometa virus, goose parvovirus, granulosis virus, Gross' virus, ground squirrel hepatitis B virus, group A arbovirus, Guanarito virus, guinea pig cytomegalovirus, guinea pig type C virus, Hantaan virus, Hantavirus, hard clam reovirus, hare fibroma virus, HCMV (human cytomegalovirus), hemadsorption virus 2, hemagglutinating virus of Japan, hemorrhagic fever virus, hendra virus, Henipaviruses, Hepadnavirus, hepatitis A virus, hepatitis B virus group, hepatitis C virus, hepatitis D virus, hepatitis delta virus, hepatitis E virus, hepatitis F virus, hepatitis G virus, hepatitis nonA nonB virus, hepatitis virus, hepatitis virus (nonhuman), hepatoencephalomyelitis reovirus 3, Hepatovirus, heron hepatitis B virus, herpes B virus, herpes simplex virus, herpes simplex virus 1, herpes simplex virus 2, herpesvirus, herpesvirus 7, Herpesvirus ateles, Herpesvirus hominis, Herpesvirus infection, Herpesvirus saimiri, Herpesvirus suis, Herpesvirus varicellae, Highlands J virus, Hirame rhabdovirus, hog cholera virus, human adenovirus 2, human alphaherpesvirus 1, human alphaherpesvirus 2, human alphaherpesvirus 3, human B lymphotropic virus, human betaherpesvirus 5, human coronavirus, human cytomegalovirus group, human foamy virus, human gammaherpesvirus 4, human gammaherpesvirus 6, human hepatitis A virus, human herpesvirus 1 group, human herpesvirus 2 group, human herpesvirus 3 group, human herpesvirus 4 group, human herpesvirus 6, human herpesvirus 8, human immunodeficiency virus, human immunodeficiency virus 1, human immunodeficiency virus 2, human papillomavirus, human T cell leukemia virus, human T cell leukemia virus I, human T cell leukemia virus II, human T cell leukemia virus III, human T cell lymphoma virus I, human T cell lymphoma virus II, human T cell lymphotropic virus type 1, human T cell lymphotropic virus type 2, human T lymphotropic virus I, human T lymphotropic virus II, human T lymphotropic virus III, Ichnovirus, infantile gastroenteritis virus, infectious bovine rhinotracheitis virus, infectious haematopoietic necrosis virus, infectious pancreatic necrosis virus, influenza virus A, influenza virus B, influenza virus C, influenza virus D, influenza virus pr8, insect iridescent virus, insect virus, iridovirus, Japanese B virus, Japanese encephalitis virus, JC virus, Junin virus, Kaposi's sarcoma-associated herpesvirus, Kemerovo virus, Kilham's rat virus, Klamath virus, Kolongo virus, Korean hemorrhagic fever virus, kumba virus, Kysanur forest condition virus, Kyzylagach virus, La Crosse virus, lactic dehydrogenase elevating virus, lactic dehydrogenase virus, Lagos bat virus, Langur virus, lapine parvovirus, Lassa fever virus, Lassa virus, latent rat virus, LCM virus, Leaky virus, Lentivirus, Leporipoxvirus, leukemia virus, leukovirus, lumpy skin condition virus, lymphadenopathy associated virus, Lymphocryptovirus, lymphocytic choriomeningitis virus, lymphoproliferative virus group, Machupo virus, mad itch virus, mammalian type B oncovirus group, mammalian type B retroviruses, mammalian type C retrovirus group, mammalian type D retroviruses, mammary tumor virus, Mapuera virus, Marburg virus, Marburg-like virus, Mason Pfizer monkey virus, Mastadenovirus, Mayaro virus, ME virus, measles virus, Menangle virus, Mengo virus, Mengovirus, Middelburg virus, milkers nodule virus, mink enteritis virus, minute virus of mice, MLV related virus, MM virus, Mokola virus, Molluscipoxvirus, Molluscum contagiosum virus, monkey B virus, monkeypox virus, Mononegavirales, Morbillivirus, Mount Elgon bat virus, mouse cytomegalovirus, mouse encephalomyelitis virus, mouse hepatitis virus, mouse K virus, mouse leukemia virus, mouse mammary tumor virus, mouse minute virus, mouse pneumonia virus, mouse poliomyelitis virus, mouse polyomavirus, mouse sarcoma virus, mousepox virus, Mozambique virus, Mucambo virus, mucosal condition virus, mumps virus, murid betaherpesvirus 1, murid cytomegalovirus 2, murine cytomegalovirus group, murine encephalomyelitis virus, murine hepatitis virus, murine leukemia virus, murine nodule inducing virus, murine polyomavirus, murine sarcoma virus, Muromegalovirus, Murray Valley encephalitis virus, myxoma virus, Myxovirus, Myxovirus multiforme, Myxovirus parotitidis, Nairobi sheep condition virus, Nairovirus, Nanirnavirus, Nariva virus, Ndumo virus, Neethling virus, Nelson Bay virus, neurotropic virus, New World Arenavirus, newborn pneumonitis virus, Newcastle condition virus, Nipah virus, noncytopathogenic virus, Norwalk virus, nuclear polyhedrosis virus (NPV), nipple neck virus, O'nyong'nyong virus, Ockelbo virus, oncogenic virus, oncogenic viruslike particle, oncornavirus, Orbivirus, Orf virus, Oropouche virus, Orthohepadnavirus, Orthomyxovirus, Orthopoxvirus, Orthoreovirus, Orungo, ovine papillomavirus, ovine catarrhal fever virus, owl monkey herpesvirus, Palyam virus, Papillomavirus, Papillomavirus sylvilagi, Papovavirus, parainfluenza virus, parainfluenza virus type 1, parainfluenza virus type 2, parainfluenza virus type 3, parainfluenza virus type 4, Paramyxovirus, Parapoxvirus, paravaccinia virus, Parvovirus, Parvovirus B 19, parvovirus group, Pestivirus, Phlebovirus, phocine distemper virus, Picodnavirus, Picornavirus, pig cytomegalovirus - pigeonpox virus, Piry virus, Pixuna virus, pneumonia virus of mice, Pneumovirus, poliomyelitis virus, poliovirus, Polydnavirus, polyhedral virus, polyoma virus, Polyomavirus, Polyomavirus bovis, Polyomavirus cercopitheci, Polyomavirus hominis 2, Polyomavirus maccacae 1, Polyomavirus muris 1, Polyomavirus muris 2, Polyomavirus papionis 1, Polyomavirus papionis 2, Polyomavirus sylvilagi, Pongine herpesvirus 1, porcine epidemic diarrhea virus, porcine hemagglutinating encephalomyelitis virus, porcine parvovirus, porcine transmissible gastroenteritis virus, porcine type C virus, pox virus, poxvirus, poxvirus variolae, Prospect Hill virus, Provirus, pseudocowpox virus, pseudorabies virus, psittacinepox virus, quailpox virus, rabbit fibroma virus, rabbit kidney vaculolating virus, rabbit papillomavirus, rabies virus, raccoon parvovirus, raccoonpox virus, Ranikhet virus, rat cytomegalovirus, rat parvovirus, rat virus, Rauscher's virus, recombinant vaccinia virus, recombinant virus, reovirus, reovirus 1, reovirus 2, reovirus 3, reptilian type C virus, respiratory infection virus, respiratory syncytial virus, respiratory virus, reticuloendotheliosis virus, Rhabdovirus, Rhabdovirus carpia, Rhadinovirus, Rhinovirus, Rhizidiovirus, Rift Valley fever virus, Riley's virus, rinderpest virus, RNA tumor virus, Ross River virus, Rotavirus, rougeole virus, Rous sarcoma virus, rubella virus, rubeola virus, Rubivirus, Russian autumn encephalitis virus, SA 11 simian virus, SA2 virus, Sabia virus, Sagiyama virus, Saimirine herpesvirus 1, salivary gland virus, sandfly fever virus group, Sandjimba virus, SARS virus, SDAV (sialodacryoadenitis virus), sealpox virus, Semliki Forest Virus, Seoul virus, sheeppox virus, Shope fibroma virus, Shope papilloma virus, simian foamy virus, simian hepatitis A virus, simian human immunodeficiency virus, simian immunodeficiency virus, simian parainfluenza virus, simian T cell lymphotrophic virus, simian virus, simian virus 40, Simplexvirus, Sin Nombre virus, Sindbis virus, smallpox virus, South American hemorrhagic fever viruses, sparrowpox virus, Spumavirus, squirrel fibroma virus, squirrel monkey retrovirus, SSV 1 virus group, STLV (simian T lymphotropic virus) type I, STLV (simian T lymphotropic virus) type II, STLV (simian T lymphotropic virus) type III, stomatitis papulosa virus, submaxillary virus, suid alphaherpesvirus 1, suid herpesvirus 2, Suipoxvirus, swamp fever virus, swinepox virus, Swiss mouse leukemia virus, TAC virus, Tacaribe complex virus, Tacaribe virus, Tanapox virus, Taterapox virus, Tench reovirus, Theiler's encephalomyelitis virus, Theiler's virus, Thogoto virus, Thottapalayam virus, Tick borne encephalitis virus, Tioman virus, Togavirus, Torovirus, tumor virus, Tupaia virus, turkey rhinotracheitis virus, turkeypox virus, type C retroviruses, type D oncovirus, type D retrovirus group, ulcerative condition rhabdovirus, Una virus, Uukuniemi virus group, vaccinia virus, vacuolating virus, varicella zoster virus, Varicellovirus, Varicola virus, variola major virus, variola virus, Vasin Gishu condition virus, VEE virus, Venezuelan equine encephalitis virus, Venezuelan equine encephalomyelitis virus, Venezuelan hemorrhagic fever virus, vesicular stomatitis virus, Vesiculovirus, Vilyuisk virus, viper retrovirus, viral haemorrhagic septicemia virus, Visna Maedi virus, Visna virus, volepox virus, VSV (vesicular stomatitis virus), Wallal virus, Warrego virus, wart virus, WEE virus, West Nile virus, western equine encephalitis virus, western equine encephalomyelitis virus, Whataroa virus, Winter Vomiting Virus, woodchuck hepatitis B virus, woolly monkey sarcoma virus, wound tumor virus, WRSV virus, Yaba monkey tumor virus, Yaba virus, Yatapoxvirus, yellow fever virus, and the Yug Bogdanovac virus. According to the invention, the virus is a herpesvirus.

### INFLAMMATORY CONDITIONS

Inflammatory conditions that can be treated and/or prevented using the methods and compositions disclosed herein include, for example, autoimmune condition. Autoimmune conditions include, for example, multiple sclerosis, Sjogren's syndrome, autoimmune hepatitis, autoimmune thyroiditis, hemophagocytic syndrome (hemophagocytic lymphohistiocytosis), diabetes mellitus type 1, Crohn's condition, ulcerative colitis, psoriasis, psoriatic arthritis, idiopathic thrombocytonpenic pupura, polymyositis, dermatomyositis, myasthenia gravis, autoimmune, thryroiditis, Evan's syndrome, autoimmune hemolytic anemia, aplastic anemia, autoimmune neutropenia, scleroderma, Reiter's syndrome, ankylosing spondylitis, pemphnigus, pemphigoid or autoimmune hepatitis, Behcet's condition, Celiac condition, Chagas condition, acute disseminated encephalomyelitis, Addison's condition, antiphospholipid antibody syndrome, autoimmune inner ear condition, bullous pemphigoid, Chronic obstructive pulmonary condition, Goodpasture's syndrome, Graves' condition, Guillain-Barré syndrome, Hashimoto's thyroditis, Hidradenitis suppurativa, Interstitial cystitis, neuromyotonia, pemphigus vulgaris, pernicious anemia, primary biliary cirrhosis, and vasculitis syndromes. Only the treatment or prevention of autoimmune conditions which are caused by herpesvirus infection in a subject having a latent herpesvirus infection with the claimed combination is part of the claimed invention.

Other inflammatory conditions that can be treated and/or prevented using the methods and compositions disclosed herein include, for example, an allergic condition (e.g., allergic rhinitis, asthma, atopic eczema), a skin condition, coronary artery condition, peripheral artery condition, atherosclerosis, retinitis, pancreatitis, cardiomyopathy, pericarditis, colitis, glomerulonephritis, lung inflammation, esophagitis, gastritis, duodenitis, ileitis, meningitis, encephalitis, encephalomyelitis, transverse myelitis, cystitis, urethritis, mucositis, lymphadenitis, dermatitis, hepatitis, osteomyelitis, or herpes zoster. Only the treatment or prevention of inflammatory conditions which are caused by herpesvirus infection in a subject having a latent herpesvirus infection with the claimed combination is part of the claimed invention.

### FORMULATIONS, ROUTES OF ADMINISTRATION, AND EFFECTIVE DOSES

Disclosed but not claimed herein are also formulations, routes of administration and effective doses for pharmaceutical compositions comprising an agent or combination of agents. Such pharmaceutical compositions can be used to treat a virus-induced inflammatory condition as described above. A pharmaceutical composition can comprise a viral inducing agent. A pharmaceutical composition can comprise a viral inducing agent and one or more additional agents. A pharmaceutical composition can comprise an antiviral agent. A pharmaceutical composition can comprise an antiviral agent and one or more additional agents. A pharmaceutical composition can comprise a viral inducing agent and an antiviral agent. A pharmaceutical composition can comprise a viral inducing agent, an antiviral agent, and one or more additional agents.

The agents or their pharmaceutically acceptable salts can be provided alone or in combination with one or more other agents or with one or more other forms. For example, a formulation can comprise one or more agents in particular proportions, depending on the relative potencies of each agent and the intended indication. For example, in compositions for targeting two different targets and where potencies are similar, about a 1:1 ratio of agents can be used. The two forms can be formulated together, in the same dosage unit e.g. in one cream, suppository, tablet, capsule, enteric coated tablet or capsule, aerosol spray, or packet of powder to be dissolved in a beverage; or each form may be formulated in a separate unit,
e.g., two creams, two suppositories, two tablets, two capsules, a tablet and a liquid for dissolving the tablet, two aerosol sprays, or a packet of powder and a liquid for dissolving the powder, etc.

A "pharmaceutically acceptable salt" can be a salt that retains the biological effectiveness and properties of one or more agents, and which are not biologically or otherwise undesirable. For example, a pharmaceutically acceptable salt does not interfere with the beneficial effect of a viral inducing agent or an antiviral agent.

Salts can include those of the inorganic ions, for example, sodium, potassium, calcium, magnesium ions, and the like. Salts can include salts with inorganic or organic acids, for example, hydrochloric acid, hydrobromic acid, phosphoric acid, nitric acid, sulfuric acid, methanesulfonic acid, p-toluenesulfonic acid, acetic acid, fumaric acid, succinic acid, lactic acid, mandelic acid, malic acid, citric acid, tartaric acid or maleic acid. If one or more agents contain a carboxy group or other acidic group, it can be converted into a pharmaceutically acceptable addition salt with inorganic or organic bases. Examples of suitable bases include sodium hydroxide, potassium hydroxide, ammonia, cyclohexylamine, dicyclohexyl-amine, ethanolamine, diethanolamine, triethanolamine, and the like.

A pharmaceutically acceptable ester or amide can be an ester or amide that retains biological effectiveness and properties of one or more agents, and which are not biologically or otherwise undesirable. For example, the ester or amide does not interfere with the beneficial effect of a viral inducing agent, an antiviral agent, or an additional agent. Esters can include, for example, ethyl, methyl, isobutyl, ethylene glycol, and the like. Amides include can include, for example, unsubstituted amides, alkyl amides, dialkyl amides, and the like.

A viral inducing agent, for example a HDAC inhibitor, can be administered in combination with an antiviral agent. Pharmaceutical compositions comprising a combination of a viral inducing agent and an antiviral agent can be formulated to comprise certain molar ratios. For example, molar ratios of about 99:1 to about 1:99 of a viral inducing agent to the antiviral agent can be used. The range of molar ratios of viral inducing agent: the antiviral agent can be selected from about 80:20 to about 20:80; about 75:25 to about 25:75, about 70:30 to about 30:70, about 66:33 to about 33:66, about 60:40 to about 40:60; about 50:50; and about 90:10 to about 10:90. The viral inducing agent and the antiviral agent can be co-formulated, in the same dosage unit, e.g., in one cream, suppository, tablet, capsule, enteric coated capsule or tablet, or packet of powder to be dissolved in a beverage; or each agent, form, and/or compound can be formulated in separate units, e.g., two creams, suppositories, tablets, two capsules, enteric coated capsules or tablets, a tablet and a liquid for dissolving the tablet, an aerosol spray a packet of powder and a liquid for dissolving the powder, etc.

An agent can be administered in combination with one or more other compounds, forms, and/or agents, e.g., as described above. Pharmaceutical compositions comprising combinations of a viral inducing agent and/or antiviral agent with one or more other active agents can be formulated to comprise certain molar ratios. For example, molar ratios of about 99:1 to about 1:99 of a viral inducing agent to the other active agent can be used; molar ratios of about 99:1 to about 1:99 of an antiviral agent to the other active agent can be used; molar ratios of about 99:1 to about 1:99 of a viral inducing agent and antiviral agent can be used. The range of molar ratios of viral inducing agent: other active agent can be selected from about 80:20 to about 20:80; about 75:25 to about 25:75, about 70:30 to about 30:70, about 66:33 to about 33:66, about 60:40 to about 40:60; about 50:50; and about 90:10 to about 10:90. The range of molar ratios of an antiviral agent: other active agent can be selected from about 80:20 to about 20:80; about 75:25 to about 25:75, about 70:30 to about 30:70, about 66:33 to about 33:66, about 60:40 to about 40:60; about 50:50; and about 90:10 to about 10:90. The molar ratio may of a viral inducing agent: other active agent can be about 1:9 or about 1:1. The molar ratio may of an antiviral agent: other active agent can be about 1:9 or about 1:1. Two or more agents, forms and/or compounds can be formulated together, in the same dosage unit, e.g., in one cream, suppository, tablet, capsule, enteric coated capsule or tablet, or packet of powder to be dissolved in a beverage; or each agent, form, and/or compound can be formulated in separate units, e.g., two creams, suppositories, tablets, two capsules, enteric coated capsules or tablets, a tablet and a liquid for dissolving the tablet, an aerosol spray a packet of powder and a liquid for dissolving the powder, etc.

A viral inducing agent, for example a HDAC inhibitor, can be administered in combination with an antiviral agent. Pharmaceutical compositions comprising a combination of a viral inducing agent and an antiviral agent can be formulated to comprise certain mg per dose. For example, a viral inducing agent can be administered at 0.01, 0.05, 0.1, 0.5, 1, 2, 5, 10, 20, 25, 50, 100, 250, 500, 1000 mg/kg per dose. A HDAC inhibitor can be administered at 0.01-0.1, 0.05-0.5, 1- 2, 1-5, 5-10, 10-20, 10-25, 10-50, 100-500, or 500-1000 mg/kg per dose. A single dose of an oral formulation of a viral inducing agent can contain 0.01, 0.05, 0.1, 0.5, 1, 2, 5, 10, 20, 25, 50, 100, 250, 500, 1000 mg. In one embodiment, the HDAC inhibitor is administered at 0.01, 0.05, 0.1, 0.5, 1, 2, 5, 10, 20, 25, 50, 100, 250, 500, 1000 mg/kg per dose. In a related embodiment, the HDAC inhibitor is administered orally. In a specific embodiment, the total daily oral dose of a HDAC inhibitor is no more than 1, 2, 5, 10, 20, 25, 40, 50, 100, 250, or 500 mg. In another related embodiment, the HDAC inhibitor is administered 1, 2, 3, 4, or 5 times a day orally. In other embodiments, a single daily dose of a HDAC inhibitor is provided whereas oral valganciclovir is provided at 900 mgs/dose, two times a day.

An oral formulation of an HDAC inhibitor can be co-formulated with an antiviral agent. The antiviral agent can be valganciclovir, ganciclovir, acyclovir. In certain embodiments, the HDAC inhibitor can be in a slow release or timed release form. In certain embodiments, the antiviral can be in a slow release or timed release form. In a specific embodiment when an HDAC inhibitor and valganciclovir are co-formulated for a single daily dose, the valganciclovir is present in a slow release or timed release form. In certain embodiments, the HDAC inhibitor and valganciclovir or other antiviral agent are co-formulated such that the HDAC inhibitor is present at no more than 100 mg per dose, and the antiviral agent is present at no more than 1000 mg per dose. In some embodiments, the HDAC inhibitor and valganciclovir or other antiviral agent are co-formulated such that the HDAC inhibitor is present at no more than 80 mg per dose, and the antiviral agent is present at no more than 500 mg per dose. In certain embodiments, the HDAC inhibitor and valganciclovir or other antiviral agent are co-formulated such that the HDAC inhibitor is present at not greater than 80 mg per dose, and the antiviral agent is present at not greater than 1500 mg per dose.

In some embodiments, a co-formulation comprising an HDAC inhibitor and an antiviral agent comprises less than 500 mg, less than 400 mg, less than 300 mg, less than 200 mg, less than 100 mg, less than 90 mg, less than 80 mg, less than 70 mg, less than 60 mg, less than 50 mg, less than 40 mg, less than 30 mg, less than 20 mg, less than 10 mg, less than 5 mg, less than 2 mg, or less than 1 mg of the HDAC inhibitor. In other embodiments, a co-formulation comprising an HDAC inhibitor and an antiviral agent comprises less than 1500 mg, less than 1400 mg, less than 1300 mg, less than 1200 mg, less than 1100 mg, less than 1000 mg, less than 900 mg, less than 800 mg, less than 700 mg, less than 600 mg, less than 500 mg, less than 400 mg, less than 300 mg, less than 200 mg, less than 100 mg, less than 90 mg, less than 80 mg, less than 70 mg, less than 60 mg, less than 50 mg, less than 40 mg, less than 30 mg, less than 20 mg, less than 10 mg, less than 5 mg, less than 2 mg, or less than 1 mg of the antiviral agent.

In certain embodiments, a unit dose of a co-formulated HDAC inhibitor and antiviral agent comprises between about 1 mg and about 500 mg of the HDAC inhibitor and between 1 mg and 1500 mg of the antiviral agent. In some embodiments, the unit dose comprises about 500 mg, about 400 mg, about 300 mg, about 200 mg, about 100 mg, about 90 mg, about 80 mg, about 70 mg, about 60 mg, about 50 mg, about 40 mg, about 30 mg, about 20 mg, about 10 mg, about 5 mg, about 2 mg, or about 1 mg of the HDAC inhibitor. In certain embodiments, the unit dose comprises less than 500 mg, less than 400 mg, less than 300 mg, less than 200 mg, less than 100 mg, less than 90 mg, less than 80 mg, less than 70 mg, less than 60 mg, less than 50 mg, less than 40 mg, less than 30 mg, less than 20 mg, less than 10 mg, less than 5 mg, less than 2 mg, or less than 1 mg of an HDAC inhibitor. In some embodiments, the unit dose comprises more than 500 mg, more than 400 mg, more than 300 mg, more than 200 mg, more than 100 mg, more than 90 mg, more than 80 mg, more than 70 mg, more than 60 mg, more than 50 mg, more than 40 mg, more than 30 mg, more than 20 mg, more than 10 mg, more than 5 mg, more than 2 mg, or more than 1 mg of an HDAC inhibitor. In certain embodiments, the unit dose comprises more than 2 mg and less than 500 mg of an HDAC inhibitor. In some embodiments, the unit dose comprises more than 10 mg and less than 50 mg of an HDAC inhibitor.

In some embodiments, the unit dose comprises about 2000 mg, about 1900 mg, about 1800 mg, about 1700 mg, about 1600 mg, comprises about 1500 mg, about 1400 mg, about 1300 mg, about 1200 mg, about 1100 mg, about 1000 mg, about 900 mg, about 800 mg, about 750 mg, about 700 mg, about 650 mg, about 600 mg, about 550 mg, about 500 mg, about 450 mg, about 400 mg, about 350 mg, about 300 mg, about 250 mg, about 200 mg, about 150 mg, about 140 mg, about 130 mg, about 120 mg, about 110 mg, about 100 mg, about 90 mg, about 80 mg, about 70 mg, about 60 mg, about 50 mg, about 40 mg, about 30 mg, about 20 mg, about 10 mg, about 5 mg, about 2 mg, or about 1 mg of the antiviral agent. In certain embodiments, the unit dose comprises less than 2000 mg, less than 1900 mg, less than 1800 mg, less than 1700 mg, less than 1600 mg, comprises less than 1500 mg, less than 1400 mg, less than 1300 mg, less than 1200 mg, less than 1100 mg, less than 1000 mg, less than 900 mg, less than 800 mg, less than 750 mg, less than 700 mg, less than 650 mg, less than 600 mg, less than 550 mg, less than 500 mg, less than 450 mg, less than 400 mg, less than 350 mg, less than 300 mg, less than 250 mg, less than 200 mg, less than 150 mg, less than 140 mg, less than 130 mg, less than 120 mg, less than 110 mg, less than 100 mg, less than 90 mg, less than 80 mg, less than 70 mg, less than 60 mg, less than 50 mg, less than 40 mg, less than 30 mg, less than 20 mg, less than 10 mg, less than 5 mg, less than 2 mg, or less than 1 mg of the antiviral agent. In some embodiments, the unit dose comprises more than 2000 mg, more than 1900 mg, more than 1800 mg, more than 1700 mg, more than 1600 mg, comprises more than 1500 mg, more than 1400 mg, more than 1300 mg, more than 1200 mg, more than 1100 mg, more than 1000 mg, more than 900 mg, more than 800 mg, more than 750 mg, more than 700 mg, more than 650 mg, more than 600 mg, more than 550 mg, more than 500 mg, more than 450 mg, more than 400 mg, more than 350 mg, more than 300 mg, more than 250 mg, more than 200 mg, more than 150 mg, more than 140 mg, more than 130 mg, more than 120 mg, more than 110 mg, more than 100 mg, more than 90 mg, more than 80 mg, more than 70 mg, more than 60 mg, more than 50 mg, more than 40 mg, more than 30 mg, more than 20 mg, more than 10 mg, more than 5 mg, more than 2 mg, or more than 1 mg of the antiviral agent. In certain embodiments, the unit dose comprises more than 50 mg and less than 1500 mg of the antiviral agent. In some embodiments, the unit dose comprises more than 100 mg and less than 500 mg of the antiviral agent. In certain embodiments, the antiviral agent is formulated as slow release.

In some embodiments, the co-formulated HDAC inhibitor and antiviral agent are administered once a day. In certain embodiments, the co-formulated HDAC inhibitor and antiviral agent are administered twice a day. In other embodiments, the co-formulated HDAC inhibitor and antiviral agent are administered thrice a day. In some embodiments, the co-formulated HDAC inhibitor and antiviral agent are administered once a day, twice a day, or thrice a day, and a further dose of the HDAC inhibitor is administered once, twice, or thrice a day. In certain embodiments, the co-formulated HDAC inhibitor and antiviral agent are administered once a day, twice a day, or thrice a day, and a further dose of the antiviral agent is administered once, twice, or thrice a day.

In certain embodiments, one unit dose of the co-formulated HDAC inhibitor and antiviral agent are administered per day. In some embodiments, two unit doses of the co-formulated HDAC inhibitor and antiviral agent are administered per day. In certain embodiments, three unit doses of the co-formulated HDAC inhibitor and antiviral agent are administered per day. In some embodiments, four unit doses of the co-formulated HDAC inhibitor and antiviral agent are administered per day. In certain embodiments, the one, two, three, or four unit doses are administered daily, once a week, twice a week, three times a week, four times a week, or five times a week.

In some embodiments, one or more unit doses of the co-formulated HDAC inhibitor and antiviral agent are administered in combination with other treatments, such as antibodies, chemotherapy drugs, and radiation therapy.

One or more agents and/or combinations of agents can be administered with still other agents. The choice of agents that can be co-administered with the agents and/or combinations of agents can depend, at least in part, on the condition being treated. Agents of particular use in the formulations disclosed but not claimed herein include, for example, any agent having a therapeutic effect for a virus-induced inflammatory condition, including, e.g., drugs used to treat inflammatory conditions. For example, formulations disclosed herein can additionally contain one or more conventional anti-inflammatory drugs, such as an NSAID, e.g. ibuprofen, naproxen, acetominophen, ketoprofen, or aspirin. For the treatment of a virus-induced inflammatory condition can additionally contain one or more conventional influenza antiviral agents, such as amantadine, rimantadine, zanamivir, and oseltamivir. In treatments for retroviral infections, such as HIV, formulations disclosed herein may additionally contain one or more conventional antiviral drug, such as protease inhibitors (lopinavir/ritonavir {Kaletra^{™}}, indinavir {Crixivan^{™}}, ritonavir {Norvir^{™}}, nelfinavir {Viracept^{™}}, saquinavir hard gel capsules {Invirase^{™}}, atazanavir {Reyataz^{™}}, amprenavir {Agenerase^{™}}, fosamprenavir {Telzir^{™}}, tipranavir{Aptivus^{™}}), reverse transcriptase inhibitors, includingnon-Nucleoside and Nucleoside/nucleotide inhibitors(AZT {zidovudine, Retrovir^{™}},ddI {didanosine, Videx^{™}}, 3TC {lamivudine, Epivir^{™}}, d4T {stavudine, Zerit^{™}}, abacavir {Ziagen^{™}}, FTC {emtricitabine, Emtriva^{™}}, tenofovir {Viread^{™}}, efavirenz {Sustiva^{™}} and nevirapine {Viramune^{™}}), fusion inhibitors T20 {enfuvirtide, Fuzeon^{™}}, integrase inhibitors (MK-0518 and GS-9137), and maturation inhibitors (PA-457 {Bevirimat^{™}}). As another example, formulations can additionally contain one or more supplements, such as vitamin C, E or other anti-oxidants.

One or more agents (or pharmaceutically acceptable salts, esters or amides thereof) can be administered per se or in the form of a pharmaceutical composition wherein the one or more active agent(s) is in an admixture or mixture with one or more pharmaceutically acceptable carriers. A pharmaceutical composition, as used herein, can be any composition prepared for administration to a subject. Pharmaceutical compositions can be formulated in conventional manner using one or more physiologically acceptable carriers, comprising excipients, diluents, and/or auxiliaries, e.g., that facilitate processing of the active agents into preparations that can be administered. Proper formulation can depend at least in part upon the route of administration chosen. One or more agents, or pharmaceutically acceptable salts, esters, or amides thereof, can be delivered to a patient using a number of routes or modes of administration, including oral, buccal, topical, rectal, transdermal, transmucosal, subcutaneous, intravenous, and intramuscular applications, as well as by inhalation.

For oral administration, one or more agents can be formulated readily by combining the one or more active agents with pharmaceutically acceptable carriers well known in the art. Such carriers can enable the one or more agents to be formulated as tablets, including chewable tablets, pills, dragees, capsules, lozenges, hard candy, liquids, gels, syrups, slurries, powders, suspensions, elixirs, wafers, and the like, for oral ingestion by a patient to be treated. Such formulations can comprise pharmaceutically acceptable carriers including solid diluents or fillers, sterile aqueous media and various non-toxic organic solvents. Generally, the agents disclosed herein can be included at concentration levels ranging from about 0.5%, about 5%, about 10%, about 20%, or about 30% to about 50%, about 60%, about 70%, about 80% or about 90% by weight of the total composition of oral dosage forms, in an amount sufficient to provide a desired unit of dosage.

Aqueous suspensions for oral use can contain one or more agents with pharmaceutically acceptable excipients, such as a suspending agent (e.g., methyl cellulose), a wetting agent (e.g., lecithin, lysolecithin and/or a long-chain fatty alcohol), as well as coloring agents, preservatives, flavoring agents, and the like.

Oils or non-aqueous solvents can be required to bring one or more agents into solution, due to, for example, the presence of large lipophilic moieties. Alternatively, emulsions, suspensions, or other preparations, for example, liposomal preparations, can be used. With respect to liposomal preparations, any known methods for preparing liposomes for treatment of a condition can be used. See, for example, Bangham et al., J. Mol. Biol. 23: 238-252 (1965) and Szoka et al., Proc. Natl Acad. Sci. USA 75: 4194-4198 (1978). Ligands can also be attached to the liposomes to direct these compositions to particular sites of action. One or more agents can also be integrated into foodstuffs, e.g, cream cheese, butter, salad dressing, or ice cream to facilitate solubilization, administration, and/or compliance in certain patient populations.

Pharmaceutical preparations for oral use can be obtained as a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; flavoring elements, cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinyl pyrrolidone (PVP). Disintegrating agents can be added, for example, the cross-linked polyvinyl pyrrolidone, agar, or alginic acid or a salt thereof such as sodium alginate. One or more agents can also be formulated as a sustained release preparation.

Dragee cores can be provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of one or more active agents.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active agents can be dissolved or suspended in suitable liquids, such as fatty oils, liquid paraffin, or liquid polyethylene glycols. In addition, stabilizers can be added. All formulations for oral administration can be in dosages suitable for administration.

For injection, one or more agents can be formulated in aqueous solutions, including but not limited to physiologically compatible buffers such as Hank's solution, Ringer's solution, or physiological saline buffer. Such compositions can also include one or more excipients, for example, preservatives, solubilizers, fillers, lubricants, stabilizers, albumin, and the like. Methods of formulation are known in the art, for example, as disclosed in Remington's Pharmaceutical Sciences, latest edition, Mack Publishing Co., Easton P.

One or more agents can also be formulated as a depot preparation. Such long acting formulations can be administered by implantation or transcutaneous delivery (for example subcutaneously or intramuscularly), intramuscular injection or use of a transdermal patch. Thus, for example, one or more agents can be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising one or more agents can exert local and regional effects when administered topically or injected at or near particular sites of infection. Direct topical application, e.g., of a viscous liquid, gel, jelly, cream, lotion, ointment, suppository, foam, or aerosol spray, can be used for local administration, to produce, for example local and/or regional effects. Pharmaceutically appropriate vehicles for such formulation include, for example, lower aliphatic alcohols, polyglycols (e.g., glycerol or polyethylene glycol), esters of fatty acids, oils, fats, silicones, and the like. Such preparations may also include preservatives (e.g., p-hydroxybenzoic acid esters) and/or antioxidants (e.g., ascorbic acid and tocopherol). *See also* Dermatological Formulations: Percutaneous absorption, Barry (Ed.), Marcel Dekker Incl, 1983. Local/topical formulations comprising a viral inducing agent and or antiviral agent may be used to treat epidermal or mucosal viral-induced inflammatory condition.

Pharmaceutical compositions can contain a cosmetically or dermatologically acceptable carrier. Such carriers can be compatible with skin, nails, mucous membranes, tissues and/or hair, and can include any conventionally used cosmetic or dermatological carrier meeting these requirements. Such carriers can be readily selected by one of ordinary skill in the art. In formulating skin ointments, an agent or combination of agents can be formulated in an oleaginous hydrocarbon base, an anhydrous absorption base, a water-in-oil absorption base, an oil-in-water water-removable base and/or a water-soluble base.

The compositions disclosed herein can be in any form suitable for topical application, including aqueous, aqueous-alcoholic or oily solutions, lotion or serum dispersions, aqueous, anhydrous or oily gels, emulsions obtained by dispersion of a fatty phase in an aqueous phase (O/W or oil in water) or, conversely, (W/O or water in oil), microemulsions or alternatively microcapsules, microparticles or lipid vesicle dispersions of ionic and/or nonionic type. These compositions can be prepared according to conventional methods. The amounts of the various constituents of the compositions disclosed herein can be those conventionally used in the art. These compositions constitute protection, treatment or care creams, milks, lotions, gels or foams for the face, for the hands, for the body and/or for the mucous membranes, or for cleansing the skin. The compositions can also consist of solid preparations constituting soaps or cleansing bars.

A pharmaceutical composition can also contain adjuvants common to the cosmetic and dermatological fields, for example, hydrophilic or lipophilic gelling agents, hydrophilic or lipophilic active agents, preserving agents, antioxidants, solvents, fragrances, fillers, sunscreens, odor-absorbers and dyestuffs. The amounts of these various adjuvants can be those conventionally used in the fields considered and, for example, are from about 0.01% to about 20% of the total weight of the composition. Depending on their nature, these adjuvants can be introduced into the fatty phase, into the aqueous phase and/or into the lipid vesicles.

Ocular viral infections can be effectively treated with ophthalmic solutions, suspensions, ointments or inserts comprising an agent or combination of agents of the present disclosed herein.

Viral infections of the ear can be effectively treated with otic solutions, suspensions, ointments or inserts comprising an agent or combination of agents of the disclosed herein.

One or more agents can be delivered in soluble rather than suspension form, which can allow for more rapid and quantitative absorption to the sites of action. In general, formulations such as jellies, creams, lotions, suppositories and ointments can provide an area with more extended exposure to the agents disclosed herein, while formulations in solution, e.g., sprays, provide more immediate, short-term exposure.

Relating to topical/local application, a pharmaceutical composition can include one or more penetration enhancers. For example, the formulations can comprise suitable solid or gel phase carriers or excipients that increase penetration or help delivery of agents or combinations of agents disclosed herein across a permeability barrier, e.g., the skin. Many of these penetration-enhancing compounds are known in the art of topical formulation, and include, e.g., water, alcohols (e.g., terpenes like methanol, ethanol, 2-propanol), sulfoxides (e.g., dimethyl sulfoxide, decylmethyl sulfoxide, tetradecylmethyl sulfoxide), pyrrolidones (e.g., 2-pyrrolidone, N-methyl-2-pyrrolidone, N-(2-hydroxyethyl)pyrrolidone), laurocapram, acetone, dimethylacetamide, dimethylformamide, tetrahydrofurfuryl alcohol, L-α-amino acids, anionic, cationic, amphoteric or nonionic surfactants (e.g., isopropyl myristate and sodium lauryl sulfate), fatty acids, fatty alcohols (e.g., oleic acid), amines, amides, clofibric acid amides, hexamethylene lauramide, proteolytic enzymes, α-bisabolol, d-limonene, urea and N,N-diethyl-m-toluamide, and the like. Additional examples include humectants (e.g., urea), glycols (e.g., propylene glycol and polyethylene glycol), glycerol monolaurate, alkanes, alkanols, ORGELASE, calcium carbonate, calcium phosphate, various sugars, starches, cellulose derivatives, gelatin, and/or other polymers. A pharmaceutical composition can include one or more such penetration enhancers.

A pharmaceutical composition for local/topical application can include one or more antimicrobial preservatives, for example, quaternary ammonium compounds, organic mercurials, p-hydroxy benzoates, aromatic alcohols, chlorobutanol, and the like.

Gastrointestinal viral infections can be effectively treated with orally- or rectally delivered solutions, suspensions, ointments, enemas and/or suppositories comprising an agent or combination of agents disclosed herein.

Respiratory viral infections can be effectively treated with aerosol solutions, suspensions or dry powders comprising an agent or combination of agents of the present disclosed hereein. Administration by inhalation is particularly useful in treating viral infections of the lung, such as influenza. The aerosol can be administered through the respiratory system or nasal passages. For example, one skilled in the art will recognize that a composition disclosed herein can be suspended or dissolved in an appropriate carrier, e.g., a pharmaceutically acceptable propellant, and administered directly into the lungs using a nasal spray or inhalant. For example, an aerosol formulation comprising a viral inducing agent and/or antiviral agent can be dissolved, suspended or emulsified in a propellant or a mixture of solvent and propellant, e.g., for administration as a nasal spray or inhalant. Aerosol formulations may contain any acceptable propellant under pressure, such as a cosmetically or dermatologically or pharmaceutically acceptable propellant, as conventionally used in the art.

An aerosol formulation for nasal administration is generally an aqueous solution designed to be administered to the nasal passages in drops or sprays. Nasal solutions can be similar to nasal secretions in that they are generally isotonic and slightly buffered to maintain a pH of about 5.5 to about 6.5, although pH values outside of this range can additionally be used. Antimicrobial agents or preservatives can also be included in the formulation.

An aerosol formulation for inhalations and inhalants can be designed so that an agent or combination of agents can be carried into the respiratory tree of the subject when administered by the nasal or oral respiratory route. Inhalation solutions can be administered, for example, by a nebulizer. Inhalations or insufflations, comprising finely powdered or liquid drugs, can be delivered to the respiratory system as a pharmaceutical aerosol of a solution or suspension of the agent or combination of agents in a propellant, e.g., to aid in disbursement. Propellants can be liquefied gases, including halocarbons, for example, fluorocarbons such as fluorinated chlorinated hydrocarbons, hydrochlorofluorocarbons, and hydrochlorocarbons, as well as hydrocarbons and hydrocarbon ethers.

Halocarbon propellants can include fluorocarbon propellants in which all hydrogens are replaced with fluorine, chlorofluorocarbon propellants in which all hydrogens are replaced with chlorine and at least one fluorine, hydrogen-containing fluorocarbon propellants, and hydrogen-containing chlorofluorocarbon propellants. Halocarbon propellants are described in Johnson, U.S. Pat. No. 5,376,359, issued Dec. 27, 1994; Byron et al., U.S. Pat. No. 5,190,029, issued Mar. 2, 1993; and Purewal et al., U.S. Pat. No. 5,776,434, issued Jul. 7, 1998. Hydrocarbon propellants useful in the invention include, for example, propane, isobutane, n-butane, pentane, isopentane and neopentane. A blend of hydrocarbons can also be used as a propellant. Ether propellants include, for example, dimethyl ether as well as the ethers. An aerosol formulation disclosed herein can also comprise more than one propellant. For example, an aerosol formulation can comprise more than one propellant from the same class, such as two or more fluorocarbons; or more than one, more than two, more than three propellants from different classes, such as a fluorohydrocarbon and a hydrocarbon. Pharmaceutical compositions of disclosed herein can also be dispensed with a compressed gas, e.g., an inert gas such as carbon dioxide, nitrous oxide or nitrogen.

Aerosol formulations can also include other components, for example, ethanol, isopropanol, propylene glycol, as well as surfactants or other components such as oils and detergents. These components can serve to stabilize the formulation and/or lubricate valve components.

The aerosol formulation can be packaged under pressure and can be formulated as an aerosol using solutions, suspensions, emulsions, powders and semisolid preparations. For example, a solution aerosol formulation can comprise a solution of an agent, such as a viral inducing agent and/or antiviral agent in (substantially) pure propellant or as a mixture of propellant and solvent. The solvent can be used to dissolve the agent and/or retard the evaporation of the propellant. Solvents useful in the invention include, for example, water, ethanol and glycols. Any combination of suitable solvents can be used, optionally combined with preservatives, antioxidants, and/or other aerosol components.

An aerosol formulation can also be a dispersion or suspension. A suspension aerosol formulation may comprise a suspension of an agent or combination of agents disclosed herein, e.g., a viral inducing agent and/or antiviral agent, and a dispersing agent. Dispersing agents useful in the disclosure include, for example, sorbitan trioleate, oleyl alcohol, oleic acid, lecithin and corn oil. A suspension aerosol formulation can also include lubricants, preservatives, antioxidant, and/or other aerosol components.

An aerosol formulation can be formulated as an emulsion. An emulsion aerosol formulation can include, for example, an alcohol such as ethanol, a surfactant, water and a propellant, as well as an agent or combination of agents, e.g., a viral inducing agent and/or an antiviral agent. The surfactant used can be nonionic, anionic or cationic. One example of an emulsion aerosol formulation comprises, for example, ethanol, surfactant, water and propellant. Another example of an emulsion aerosol formulation comprises, for example, vegetable oil, glyceryl monostearate and propane.

Pharmaceutical compositions suitable for use in the present invention can include compositions wherein the active ingredients are present in an effective amount, i.e., in an amount effective to achieve therapeutic and/or prophylactic benefit in a host with at least one virus-induced inflammatory condition. The actual amount effective for a particular application will depend on the condition or conditions being treated, the condition of the subject, the formulation, and the route of administration, as well as other factors known to those of skill in the art. Determination of an effective amount of a viral inducing agent and/or antiviral agent is well within the capabilities of those skilled in the art, in light of the disclosure herein, and can be determined using routine optimization techniques.

An effective amount for use in humans can be determined from animal models. For example, a dose for humans can be formulated to achieve circulating, liver, topical and/or gastrointestinal concentrations that have been found to be effective in animals. One skilled in the art can determine the effective amount for human use, especially in light of the animal model experimental data described herein. Based on animal data, and other types of similar data, those skilled in the art can determine an effective amount of a composition appropriate for humans.

An effective amount when referring to an agent or combination of agents of the invention can generally mean the dose ranges, modes of administration, formulations, etc., that have been recommended or approved by any of the various regulatory or advisory organizations in the medical or pharmaceutical arts (e.g., FDA, AMA) or by the manufacturer or supplier.

Further, appropriate doses for a viral inducing agent and/or antiviral agent can be determined based on in vitro experimental results.

A person of skill in the art would be able to monitor in a patient the effect of administration of a particular agent. For example, HIV or EBV viral load levels can be determined by techniques standard in the art, such as measuring CD4 cell counts, and/or viral levels as detected by PCR. Other techniques would be apparent to one of skill in the art.

In a non-claimed aspect, this disclosure provides for a kit, the kits can comprise one or more containers, the kit can comprise any combination of HDAC inhibitors, antivirals or additional agents mentioned in this disclosure in suitable packaging. The kit may contain instructions for use. The HDAC inhibitor or antiviral can be present in any concentration disclosed herein, can be packaged for administration by any route disclosed herein, or in any formulation disclosed herein. The HDAC and antiviral agent may be packaged together, in a suitable package or container, in a kit. The kit may be for convenient administration or dosing, and management thereof. The HDAC inhibitor and antiviral may be formulated together as a pharmaceutical composition in a single dose. The HDAC inhibitor and antiviral may be formulated as separate pharmaceutical compositions. The pharmaceutical composition of the HDAC inhibitor may be packaged for once a week, twice a week, thrice a week, four times a week or more, once a month, twice a month, thrice a month, four times a month or more dosing; and the pharmaceutical composition of the antiviral is packaged for daily, twice daily, thrice daily, four times a day or more dosing. The antiviral is administered or taken without the HDAC inhibitor. The treatment course of the HDAC inhibitor and antiviral can be as follows: the HDAC inhibitor and the antiviral are taken or administered together in the same pharmaceutical composition on any of the first, second, third, fourth, fifth or more days of treatment; and the antiviral is taken or administered by itself on any of days 2, 3, 4, 5, 6, 7, 8, 9, 10, 11, 12, 13 or more. The treatment course can be as follows: the HDAC inhibitor and antiviral are taken or administered separately in different pharmaceutical composition on any of the first, second, third, fourth, fifth or more days of treatment, either at the same time or temporally separated by 1, 2, 3, 4, 5, 6, 7, 8, 9, 10, 11, 12, or more hours; and the antiviral is taken or administered by itself on any of days 2, 3, 4, 5, 6, 7, 8, 9, 10, 11, 12, 13 or more. The HDAC inhibitor packaged in the kit is chidamide; in other non-claimed aspects, it may be 4SC-202. The antiviral may be ganciclovir, in other embodiments, it may be valganciclovir. The treatment course may be repeated for 1, 2, 3, 4, 5, 6, 7, 8, 9, 10, 11, 12, 13, 14 or more iterations.

The kits disclosed herein may be in suitable packaging. Suitable packaging includes, but is not limited to, vials, bottles, jars, flexible packaging (e.g., sealed Mylar, blister packs, plastic bags), and the like. Also contemplated are packages for use in combination with a specific device, such as an inhaler, nasal administration device (e.g., an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is an HDAC inhibitor. The HDAC inhibitor can be 4SC-202 (not claimed) or chidamide. The container may further comprise a second pharmaceutically active agent. This second pharmaceutically active agent can be an antiviral. The antiviral can be ganciclovir or valganciclovir.

### ADMINISTRATION SCHEDULE

Administration of one or more agents (e.g, a viral inducing agent and/or an antiviral) can be intermittent; for example, administration can be once every two days, every three days, every five days, once a week, once or twice a month, and the like. The amount, forms, and/or amounts of the different forms can be varied at different times of administration.

Pulsed administration of one or more pharmaceutical compositions can be used for the treatment or prevention of a viral-induced inflammatory condition. Pulsed administration can be more effective than continuous treatment as pulsed doses can be lower than would be expected from continuous administration of the same composition. Each pulse dose can be reduced and the total amount of drug administered over the course of treatment to the patient can be minimized.

With pulse therapy, *in vivo* levels of an agent can drop below that level required for effective continuous treatment. Pulsed administration can reduce the amount of the composition administered to the patient per dose or per total treatment regimen with an increased effectiveness. Pulsed administration can provide a saving in time, effort and expense and a lower effective dose can lessen the number and severity of complications that can be experienced by a subject. As such, pulsing can be more effective than continuous administration of the same composition.

Individual pulses can be delivered to a subject continuously over a period of several hours, such as about 2, 4, 6, 8, 10, 12, 14 or 16 hours, or several days, such as 2, 3, 4, 5, 6, or 7 days, or from about 1 hour to about 24 hours or from about 3 hours to about 9 hours. Alternatively, periodic doses can be administered in a single bolus or a small number of injections of the composition over a short period of time, for example, less than 1 or 2 hours. For example, the HDAC inhibitor can be administered over a period of 14 days, followed by a period of 7 days of no treatment. For example, 4SC-202 can be administered over a period of 14 days, followed by a period of 7 days of no treatment. For example, chidamide can be administered over a period of 14 days, followed by a period of 7 days of no treatment.

The interval between pulses or the interval of no delivery can be greater than 24 hours or can be greater than 48 hours, and can be for even longer such as for 3, 4, 5, 6, 7, 8, 9 or 10 days, two, three or four weeks or even longer. The interval between pulses can be determined by one of ordinary skill in the art. The interval between pulses can be calculated by administering another dose of the composition when the composition or the active component of the composition is no longer detectable in the patient prior to delivery of the next pulse. Intervals can also be calculated from the *in* vivo half-life of the composition. Intervals can be calculated as greater than the in vivo half-life, or 2, 3, 4, 5 and even 10 times greater than the composition half-life. Intervals can be 25, 50, 100, 150, 200, 250 300 and even 500 times the half-life of the chemical composition.

The number of pulses in a single therapeutic regimen can be as little as two, but can be from about 5 to 10, 10 to 20, 15 to 30 or more. Subjects (e.g., patients) can receive one or more agents (e.g., drugs) for life according to the methods disclosed herein. Compositions can be administered by most any means, and can be delivered to the patient as an injection (e.g. intravenous, subcutaneous, intra-arterial), infusion or instillation, and more preferably by oral ingestion. Various methods and apparatus for pulsing compositions by infusion or other forms of delivery to the patient are disclosed in U.S. Pat. Nos. 4,747,825; 4,723,958; 4,948,592; 4,965,251 and 5,403,590.

In certain embodiments, the co-formulated unit dose comprising an HDAC inhibitor and an antiviral agent is administered daily. In further embodiments, administration is continuous. In some embodiments, the administration of the co-formulated unit dose is by pulsed administration. In certain embodiments, pulsed administration comprises administering pulses of the co-formulated unit dose for about 1 day, about 2 days, about 3 days, about 4 days, about 5 days, about 6 days, about 7 days, about 10 days, about 2 weeks, about 3 weeks, about 4 weeks, about 5 weeks, about 6 weeks, about 7 weeks, about 8 weeks, about 2 months, about 3 months, about 4 months, about 5 months, about 6 months, about 9 months, about 12 months. In some embodiments, pulsed administration comprises intervals of not administering the co-formulated unit dose of about 1 day, about 2 days, about 3 days, about 4 days, about 5 days, about 6 days, about 7 days, about 10 days, about 2 weeks, about 3 weeks, about 4 weeks, about 5 weeks, about 6 weeks, about 7 weeks, about 8 weeks, about 2 months, about 3 months, about 4 months, about 5 months, about 6 months, about 9 months, about 12 months.

In some embodiments, the administration of the co-formulated unit dose is by pulsed administration. In certain embodiments, the pulsed administration comprises administering the co-formulated unit dose for about 8 weeks, followed by not administering the co-formulated unit dose for about 4 weeks. In some embodiments, the pulsed administration comprises administering the co-formulated unit dose for about 6 weeks, followed by not administering the co-formulated unit dose for about 2 weeks. In certain embodiments, the pulsed administration comprises administering the co-formulated unit dose for about 4 weeks, followed by not administering the co-formulated unit dose for about 2 weeks. In some embodiments, the pulsed administration comprises administering the co-formulated unit dose for about 2 weeks, followed by not administering the co-formulated unit dose for about 2 weeks. In some embodiments, pulsed administration comprises pulses of administering the co-formulated unit dose for about 1 day, about 2 days, about 3 days, about 4 days, about 5 days, about 6 days, about 7 days, about 10 days, about 2 weeks, about 3 weeks, about 4 weeks, about 5 weeks, about 6 weeks, about 7 weeks, about 8 weeks, about 2 months, about 3 months, about 4 months, about 5 months, about 6 months, about 9 months, about 12 months. In certain embodiments, pulsed administration comprises intervals of not administering the co-formulated unit dose of about 1 day, about 2 days, about 3 days, about 4 days, about 5 days, about 6 days, about 7 days, about 10 days, about 2 weeks, about 3 weeks, about 4 weeks, about 5 weeks, about 6 weeks, about 7 weeks, about 8 weeks, about 2 months, about 3 months, about 4 months, about 5 months, about 6 months, about 9 months, about 12 months. In some embodiments, administration is continuous. In certain embodiments, administration is for the lifetime of the subject. In other embodiments, administration is for about 1 week, about 2 weeks, about 3 weeks, about 4 weeks, about 5 weeks, about 6 weeks, about 7 weeks, about 8 weeks, about 2 months, about 3 months, about 4 months, about 5 months, about 6 months, about 9 months, or about 12 months. In some embodiments, an antiviral agent is administered during intervals of not administering the co-formulated unit dose. In certain embodiments, an antiviral agent is administered in addition to the co-formulated unit dose. In some embodiments, an antiviral agent is administered simultaneously with the co-formulated unit dose. In other embodiments, an antiviral agent is administered separate from the co-formulated unit dose.

A pharmaceutical composition comprising a viral inducing agent can be administered to a subject before a pharmaceutical composition comprising an antiviral agent is administered to the subject. A pharmaceutical composition comprising a viral inducing agent can be co-administered to a subject with a pharmaceutical composition comprising an antiviral agent. A pharmaceutical composition comprising a viral inducing agent can be co-administered with a pharmaceutical composition comprising an antiviral agent and a pharmaceutical composition comprising one or more addition agents. The pharmaceutical compositions can be provided by pulsed administration. For example, a pharmaceutical composition comprising a viral inducing agent can be administered to a subject, followed by administration of a pharmaceutical composition comprising an antiviral agent to the subject after an interval of time has passed, and this order of administration the same or similar time interval can be repeated, for example, at least 1, 2, 3, 4, 5, 6, 7, 8, 9, or 10 or more times.

### EXAMPLES

### Example 1: Analysis of Efficacy of Chidamide

To measure whether Chidamide could induce EBV associated transcripts, the induction of mRNA for the protein kinases TK **(****Figure 1A****)** and BGLF4 **(****Figure 1B****)** in EBV-positive lymphoma cell lines was measured after exposure to different concentrations of the short-chain fatty acid, sodium butyrate (NaB), or the benzamide, chidamide (chid), for 24 hrs. Transcripts were quantitated by quantitative real-time RT-PCR.

To determine tumor cytotoxic activity of the combination of HDACi and GCV, EBV+ lymphoma cells were exposed to a range of concentrations of sodium butyrate or chidamide (GCV) for 3 days and then to GCV alone for another 3 days **(****Figure 1C****).** Efficacy of either sodium butyrate or chidamide in the combination treatment approach was then determined by enumerating living cells. A general experimental protocol is described below.

### Cells

The EBV-positive B lymphoma cell line P3HR1 was used in the study. The P3HR1 cell line was originally derived from Burkitt's lymphoma patient. EBV maintains a latent state of replication in this cell line. These cells were maintained in RPMI 1640 with 10% fetal bovine serum containing 100 U penicillin per ml and 100 µg streptomycin per ml.

### Study Agent

The HDAC inhibitor chidamide was used in this study. As a positive control, the Short Chain Fatty Acid butyrate, an established inducer of EBV-TK, was used. Ganciclovir (GCV) was used as the anti-viral drug.

### Titration of Chidamide on P3HR1 Cells:

Concentrations chidamide, which do not significantly affect the viability or proliferation of P3HR1 cells in culture were established.

### Drug Sensitivity Assay

To test the sensitivity of EBV-positive lymphoma cells towards chidamide, P3HR1 cells were treated with chidamide in the presence of one anti-viral drug. At the end of the assay, the efficacy of chidamide was assessed by measuring the inhibition of cell growth compared to untreated cells.

Healthy, actively-growing P3HR1 cells were harvested and resuspended in fresh growth media. Cells were seeded in wells. Appropriate dilutions of chidamide were added to certain wells, some of which received an anti-viral drug (such as GCV at 50 µM concentration). At 72 hrs., 800 µl of culture fluid was removed from each well. Wells were then refed with 1.0 ml fresh growth media without HDAC inhibitors. Fresh GCV solution was added to the wells that originally received GCV at the same initial concentration. On day 6, cell morphology was observed under a microscope and viable cell counts in each individual well was determined by the trypan blue dye exclusion method using an automated cell counter (Countess, Invitrogen).

### Thymidine Kinase and Protein Kinase Transcript Expression Assays

A Thymidine Kinase (TK) mRNA assay was used to determine if HDAC inhibitors induced TK or PK (BGLF4) expression in EBV-infected lymphoma cells.

P3HR1 cells were seeded in 60 mm plates containing 3 X 106 cells in 3 ml of fresh growth media. Appropriate concentrations of HDAC inhibitors were added to the plate and cells were incubated in the presence of HDAC inhibitors for 6h, 24h, 48h, or as needed. Cells were harvested by centrifugation and washed once in cold PBS. Total cellular RNA was then extracted. To measure the relative level of TK mRNA in various total RNA preparations, reverse transcription and quantitative PCR using real time PCR analysis were used. *See,* Ghosh, S.K., Forman, L.W., Akinsheye, I., Perrine, S.P., Faller, D.V.: Short discontinuous sodium butyrate exposure efficiently sensitizes latently EBV infected cells towards nucleoside analogue-mediated growth inhibition, Blood Cells Mol. Diseases (2007), 38:57-65. The relative level of TK expression in a sample was calculated following normalization of β-actin expression level.

### Results

The HDAC inhibitor chidamide had synergistic activity with the anti-viral agent ganciclovir in killing EBV+ lymphoma cells. Chidamide induced expression of viral TK and BGLF4 beginning at a concentration of 500 nM and was clearly superior to 1. 0 mM sodium butyrate at all concentrations tested (Figure 1A and 1B. With respect to killing, when combined with ganciclovir, 1µM of chidamide was as good as 1.0 mM sodium butyrate, when combined with ganciclovir.

### Example 2: Analysis of Efficacy of an Inducing Agent on EBV, CMV and HHV-6 Viral Levels (Reference example)

A female patient being treated for post-transplant EBV positive lymphoma with arginine butyrate (not claimed) and daily ganciclovir exhibited a significant decrease in levels of EBV, CMV and HHV-6 levels. Levels were determined before treatment (week1) and after 3 weeks of treatment (week 3). Results are shown below in Table 1.

**Table 1: Decline in viral load of multiple viruses in patient with EBV lymphoma and concurrent symptomatic viremia with CMV and HHV-6.**

| | Copies per mL | |
|---|---|---|
| **Virus** | **Week 1** | **Week 3** |
| EBV | 27,782 | 1,486 |
| CMV | 12,300 | 1,600 |
| HHV-6 | 658,882 | 805 |

### Example 3. Phase 1 Study of Chidamide Plus Valganciclovir in Patients with EBV Associated Lymphoid Malignancies

Patients with EBV-associated lymphoid malignancies, who have histologically confirmed lymphoid neoplasms that are EBV+, are treated with chidamide and valganciclovir. Valganciclovir administration is continued throughout the cycle. Chidamide is administered once a week. A dose escalation is conducted until a maximum tolerated dose is established:

Patients are evaluated for an anti-tumor response. A complete response (CR) is defined as disappearance of detectable malignant disease on imaging or physical examination (e.g., for skin lesions or tonsillar masses). A partial response (PR) is defined as a 50% decrease in tumor size (the sum of the product of the largest perpendicular diameters) or measurable lesions chosen for analysis prior to beginning of treatment. For lesions which can only be measured in 1 dimension, such as skin (cutaneous T cell lymphoma), a greater than 50% decrease in the largest dimension qualifies as a PR.

### Example 4. Phase II Trial of Chidamide or 4SC-202 and Valganciclovir in EBV(+) Lymphoid Malignancies

A clinical trial is instituted for patients with EBV-associated lymphoid malignancies, who have histologically confirmed lymphoid neoplasms that are EBV+, utilizing a once-a-week administration of chidamide or 4SC-202 (not claimed) and 21 days continuous administration of valganciclovir. Patients are monitored for response by FDG-PET and classified as a complete response (CR), partial response (PR), stable disease (SD), or progressive disease (PD).

## Claims

1. An inducing agent in combination with an antiviral agent, for use in a method for treating or preventing a cancer, autoimmune, or inflammatory condition caused by herpesvirus infection in a subject having a latent herpesvirus infection, wherein said antiviral agent is a nucleoside analog and said inducing agent is chidamide.

2. The inducing agent in combination with an antiviral agent for the use of claim 1, wherein said cancer is a cancer caused by Epstein-Barr virus infection, herpes simplex virus infection, human herpes virus 8 infection, or cytomegalovirus infection.

3. The inducing agent in combination with an antiviral agent for the use of claim 2, wherein said cancer is a lymphoma, Burkitt's Lymphoma, Hodgkin's disease, nasopharyngeal carcinoma, hairy leukoplakia, NK/T cell lymphoma, a lymphoma of the central nervous system, or mucoepidermoid carcinoma.

4. The inducing agent in combination with an antiviral agent for the use of claim 1, wherein the autoimmune or inflammatory condition is an autoimmune or inflammatory condition caused by Epstein-Barr Virus infection selected from dermatomyositis, Sjögren's syndrome, and multiple sclerosis.

5. The inducing agent in combination with an antiviral agent for the use of claim 1, wherein said inflammatory condition is caused by cytomegalovirus infection selected from hepatitis, colitis, retinitis, pneumonitis, esophagitis, transverse myelitis, encephalitis and polyradiculopathy.

6. The inducing agent in combination with an antiviral agent for the use of claim 1, wherein said cancer is a lymphoma.

7. The inducing agent in combination with an antiviral agent for the use of any one of claims 1-6 wherein the inducing agent induces expression of a viral gene product in a virus-infected cell of the subject, wherein the viral gene product is a viral enzyme, an oncogene or proto-oncogene, a transcription factor, a protease, a polymerase, a reverse transcriptase, a cell surface receptor, a structural protein, a major histocompatibility antigen, a growth factor, or a combination thereof.

8. The inducing agent in combination with an antiviral agent for the use of claim 7, wherein the viral gene product is a viral enzyme selected from a thymidine kinase (TK) or protein kinase (PK).

9. The inducing agent in combination with an antiviral agent for the use of any one of claims 1-8, wherein said inducing agent is administered at a dose of about 1.0 to about 1000 mg/day.

10. The inducing agent in combination with an antiviral agent for the use of any one of claims 1-9, wherein the nucleoside analog is selected from the group consisting of acyclovir (ACV), ganciclovir (GCV), valganciclovir, famcyclovir, penciclovir (PCV), ribavirin, zalcitabine (ddC), zidovudine (AZT), stavudine (D4T), lamivudine (3TC), didanosine (ddl), cytarabine, dideoxyadenosine, edoxudine, floxuridine, idoxuridine, inosine pranobex, 2'-deoxy-5-(methylamino)uridine, trifluridine, and vidarabine.

11. The inducing agent in combination with an antiviral agent for the use of any one of claims 1-10, wherein said inducing agent is administered orally.

12. The inducing agent in combination with an antiviral agent for the use of any one of claims 1-11, wherein said nucleoside analog is valganciclovir.

13. The inducing agent in combination with an antiviral agent for the use of any one of claims 1-11, wherein said nucleoside analog is acyclovir, ganciclovir, or valganciclovir.

## Patentansprüche

1. Induzierendes Mittel in Kombination mit einem antiviralen Mittel zur Verwendung in einem Verfahren zum Behandeln oder Vorbeugen eines durch eine Herpesvirusinfektion verursachten Krebs-, Autoimmun- oder Entzündungsleidens bei einem Subjekt mit einer latenten Herpesvirusinfektion, wobei das antivirale Mittel ein Nukleosid-Analogon ist und das induzierende Mittel Chidamid ist.

2. Induzierendes Mittel in Kombination mit einem antiviralen Mittel zur Verwendung nach Anspruch 1, wobei der Krebs ein Krebs ist, der durch eine Infektion mit dem Epstein-Barr-Virus, eine Infektion mit dem Herpes-simplex-Virus, eine Infektion mit dem humanen Herpesvirus 8 oder eine Infektion mit dem Cytomegalievirus verursacht wird.

3. Induzierendes Mittel in Kombination mit einem antiviralen Mittel zur Verwendung nach Anspruch 2, wobei der Krebs ein Lymphom, Burkitt-Lymphom, Morbus Hodgkin, Nasopharynxkarzinom, Haarleukoplakie, NK/T-Zell-Lymphom, ein Lymphom des zentralen Nervensystems oder Mukoepidermoidkarzinom ist.

4. Induzierendes Mittel in Kombination mit einem antiviralen Mittel zur Verwendung nach Anspruch 1, wobei das Autoimmun- oder Entzündungsleiden ein durch eine Infektion mit dem Epstein-Barr-Virus verursachtes Autoimmun- oder Entzündungsleiden ist, das aus Dermatomyositis, Sjögren-Syndrom und Multipler Sklerose ausgewählt ist.

5. Induzierendes Mittel in Kombination mit einem antiviralen Mittel zur Verwendung nach Anspruch 1, wobei das Entzündungsleiden durch eine Cytomegalievirusinfektion verursacht ist und aus Hepatitis, Colitis, Retinitis, Pneumonitis, Ösophagitis, transverser Myelitis, Enzephalitis und Polyradikulopathie ausgewählt ist.

6. Induzierendes Mittel in Kombination mit einem antiviralen Mittel zur Verwendung nach Anspruch 1, wobei der Krebs ein Lymphom ist.

7. Induzierendes Mittel in Kombination mit einem antiviralen Mittel zur Verwendung nach einem der Ansprüche 1-6, wobei das induzierende Mittel die Expression eines Virus-Genprodukts in einer virusinfizierten Zelle des Subjekts induziert, wobei das Virus-Genprodukt ein virales Enzym, ein Onkogen oder Proto-Onkogen, ein Transkriptionsfaktor, eine Protease, eine Polymerase, eine reverse Transkriptase, ein Zelloberflächenrezeptor, ein Strukturprotein, ein Haupthistokompatibilitätsantigen, ein Wachstumsfaktor oder eine Kombination davon ist.

8. Induzierendes Mittel in Kombination mit einem antiviralen Mittel zur Verwendung nach Anspruch 7, wobei das Virus-Genprodukt ein virales Enzym ist, das aus einer Thymidinkinase (TK) oder Proteinkinase (PK) ausgewählt ist.

9. Induzierendes Mittel in Kombination mit einem antiviralen Mittel zur Verwendung nach einem der Ansprüche 1-8, wobei das induzierende Mittel in einer Dosis von etwa 1,0 bis etwa 1000 mg/Tag verabreicht wird.

10. Induzierendes Mittel in Kombination mit einem antiviralen Mittel zur Verwendung nach einem der Ansprüche 1-9, wobei das Nukleosid-Analogon aus der Gruppe ausgewählt ist, die aus Aciclovir (ACV), Ganciclovir (GCV), Valganciclovir, Famciclovir, Penciclovir (PCV), Ribavirin, Zalcitabin (ddC), Zidovudin (AZT), Stavudin (D4T), Lamivudin (3TC), Didanosin (ddl), Cytarabin, Didesoxyadenosin, Edoxudin, Floxuridin, Idoxuridin, Inosin Pranobex, 2'-Desoxy-5-(methylamino)uridin, Trifluridin und Vidarabin besteht.

11. Induzierendes Mittel in Kombination mit einem antiviralen Mittel zur Verwendung nach einem der Ansprüche 1-10, wobei das induzierende Mittel oral verabreicht wird.

12. Induzierendes Mittel in Kombination mit einem antiviralen Mittel zur Verwendung nach einem der Ansprüche 1-11, wobei das Nukleosid-Analogon Valganciclovir ist.

13. Induzierendes Mittel in Kombination mit einem antiviralen Mittel zur Verwendung nach einem der Ansprüche 1-11, wobei das Nukleosid-Analogon Aciclovir, Ganciclovir oder Valganciclovir ist.

## Revendications

1. Agent inducteur en combinaison avec un agent antiviral, destiné à être utilisé dans un procédé de traitement ou de prévention d'un cancer, d'une affection auto-immune ou inflammatoire causée par une infection par l'herpèsvirus chez un sujet présentant une infection latente par l'herpèsvirus, ledit agent antiviral étant un analogue nucléosidique et ledit agent inducteur étant le chidamide.

2. L'agent inducteur en combinaison avec un agent antiviral destiné à être utilisé selon la revendication 1, ledit cancer étant un cancer causé par une infection par le virus d'Epstein-Barr, une infection par le virus herpès simplex, une infection par l'herpèsvirus humain 8 ou une infection par le cytomégalovirus.

3. L'agent inducteur en combinaison avec un agent antiviral destiné à être utilisé selon la revendication 2, ledit cancer étant un lymphome, le lymphome de Burkitt, la maladie de Hodgkin, un carcinome du nasopharynx, une leucoplasie chevelue, un lymphome à cellules NK/T, un lymphome du système nerveux central ou un carcinome mucoépidermoïde.

4. L'agent inducteur en combinaison avec un agent antiviral destiné à être utilisé selon la revendication 1, ladite affection auto-immune ou inflammatoire étant une affection auto-immune ou inflammatoire causée par une infection par le virus d'Epstein-Barr choisie parmi la dermatomyosite, le syndrome de Sjogren et la sclérose en plaques.

5. L'agent inducteur en combinaison avec un agent antiviral destiné à être utilisé selon la revendication 1, ladite affection inflammatoire étant causée par une infection par le cytomégalovirus choisie parmi l'hépatite, la colite, la rétinite, la pneumonie, l'oesophagite, la myélite transverse, l'encéphalite et la polyradiculopathie.

6. L'agent inducteur en combinaison avec un agent antiviral destiné à être utilisé selon la revendication 1, ledit cancer étant un lymphome.

7. L'agent inducteur en combinaison avec un agent antiviral destiné à être utilisé selon l'une quelconque des revendications 1 à 6, ledit agent inducteur induisant l'expression d'un produit génique viral dans une cellule infectée par un virus du sujet, ledit produit génique viral étant une enzyme virale, un oncogène ou un proto-oncogène, un facteur de transcription, une protéase, une polymérase, une transcriptase inverse, un récepteur de surface cellulaire, une protéine structurale, un antigène majeur d'histocompatibilité, un facteur de croissance, ou une combinaison de ceux-ci.

8. L'agent inducteur en combinaison avec un agent antiviral destiné à être utilisé selon la revendication 7, dans lequel le produit génique viral est une enzyme virale choisie parmi une thymidine kinase (TK) ou une protéine kinase (PK).

9. L'agent inducteur en combinaison avec un agent antiviral destiné à être utilisé selon l'une quelconque des revendications 1 à 8, ledit agent inducteur étant administré à une dose d'environ 1,0 à environ 1000 mg/jour.

10. L'agent inducteur en combinaison avec un agent antiviral destiné à être utilisé selon l'une quelconque des revendications 1 à 9, dans lequel l'analogue nucléosidique est choisi dans le groupe constitué de acyclovir (ACV), ganciclovir (GCV), valganciclovir, famcyclovir, penciclovir (PCV), ribavirin, zalcitabine (ddC), zidovudine (AZT), stavudine (D4T), lamivudine (3TC), didanosine (ddl), cytarabine, didésoxyadénosine, édoxudine, floxuridine, idoxuridine, inosine pranobex, 2'-désoxy-5-(méthylamino)uridine, trifluridine et vidarabine.

11. L'agent inducteur en combinaison avec un agent antiviral destiné à être utilisé selon l'une quelconque des revendications 1 à 10, ledit agent inducteur étant administré par voie orale.

12. L'agent inducteur en combinaison avec un agent antiviral destiné à être utilisé selon l'une quelconque des revendications 1 à 11, ledit analogue nucléosidique étant le valganciclovir.

13. L'agent inducteur en combinaison avec un agent antiviral destiné à être utilisé selon l'une quelconque des revendications 1 à 11, ledit analogue nucléosidique étant l'acyclovir, le ganciclovir ou le valganciclovir.
